(19) Europäisches Patentamt
European Patent Office
Office européen des brevets

(11) EP 2 020 436 A1

(12) **EUROPEAN PATENT APPLICATION**
published in accordance with Art. 153(4) EPC

(43) Date of publication:
**04.02.2009 Bulletin 2009/06**

(21) Application number: **07765843.3**

(22) Date of filing: **27.04.2007**

(51) Int Cl.:
*C12N 7/02* (2006.01) *C12N 15/861* (2006.01)

(86) International application number:
**PCT/ES2007/000260**

(87) International publication number:
**WO 2007/125146 (08.11.2007 Gazette 2007/45)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **28.04.2006 ES 200601136**

(71) Applicants:
- **Universitat Autonoma de Barcelona**
**08193 Bellaterra (ES)**
- **Institució Catalana de Recerca i Estudis Avançats**
**08010 Barcelona (ES)**

(72) Inventors:
- **CHILLÓN RODRÍGUEZ, Miguel**
**08193 Bellaterra (ES)**
- **ALBA FERNÁNDEZ, Raúl**
**08193 Bellaterra (ES)**
- **BOSCH MERINO, Assumpció**
**08193 Bellaterra (ES)**

(74) Representative: **Elzaburu Marquez, Alberto**
**Elzaburu S.A.,**
**Miguel Angel, 21**
**28010 Madrid (ES)**

(54) **METHOD FOR PRODUCING ADENOVIRUS VECTORS FOR GENE THERAPY AND DNA SEQUENCES USED THEREFOR**

(57) Method for producing adenovirus vectors for gene therapy and auxiliary vectors used therefor. The method is based on the multiplication of gutless adenoviruses that lack adenovirus-coding sequences by cotransfecting them with an auxiliary or helper adenovirus that has an attB sequence of the &phis;C31 bacteriophage inserted between the adenovirus packaging signal and the ITR closest to it and/or utilizing the delay arising at the time of packaging the helper adenovirus with respect to that of the gutless adenovirus owing to the presence of the atttB sequence in order to recover the gutless adenovirus from the culture before the helper adenovirus completes its viral cycle. This gives rise to high gutless adenovirus titres that are essentially free from helper adenovirus, thereby allowing them to be used in gene therapy, minimizing the likelihood of the appearance of a cellular immune response on the part of the treated individual against cells transduced by the adenovirus vector produced.

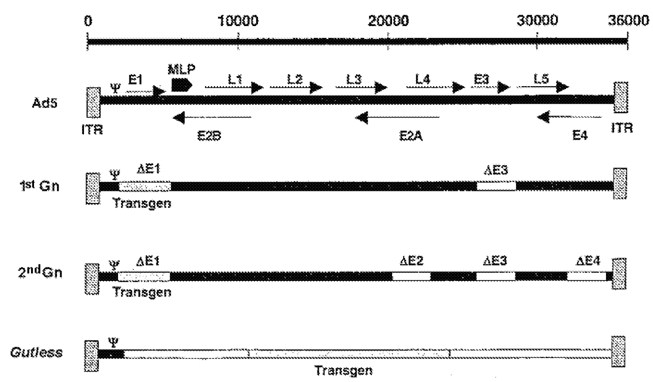


Fig. 2



Printed by Jouve, 75001 PARIS (FR)

**Description**

## TECHNICAL FIELD

The invention refers to obtaining adenovirus vectors without adenovirus coding regions (gutless adenovirus) with low contamination levels in helper adenoviruses, to allow multiplication and packaging of the vectors. The invention specifically refers to a method to produce these adenovirus without the need to use cells that express an exogenous recombinase, as well as helper adenoviruses which allow this method to be carried out.

## BACKGROUND OF THE INVENTION

Gene therapy is one of the disciplines of biomedicine that has allowed the development of techniques and protocols with therapeutic potential to treat diseases of a genetic origin. Gene therapy consists of introducing nucleic acids (DNA and RNA) for therapeutic purposes in a target cell, to permit, increase or decrease the genetic expression of a specific gene. Generally speaking treatment of the majority of genetic illnesses by somatic gene therapy requires the expression of a therapeutic gene throughout the life of the patient. Consequently the development of safe vectors that allow the expression of the gene or genes of interest, encoded by the nucleic acid introduced (the transgene) during long periods of time and which prevent an immune response by the patient is essential for the *in vivo* use of these vectors in gene therapy protocols.

At this time adenoviruses (also referred to with the abbreviation Ad) have become one of the vectors most used in clinical tests in humans, passing from 20.2% of the vectors used in these tests in 1999, to 32.2% in the 1999-2004[1] period. This increase is due to the following factors: the Ad can infect an elevated number of cell types, both quiescent cells as well as cells in division; current techniques used to produce Ad allow its capsid to be modified, and therefore its cell tropism; they are easy to produce at high titres (up to $10^{13}$ particles/milliliter); they are non-integrative viruses, therefore avoiding insertional mutagenesis. The fundamental disadvantage of the adenoviral vectors is that a high dosis-dependent immunity is induced, both humoral, making future readministraciones difficult, as well as cellular by T lymphocytes (CTL).

The adenoviruses (Ad) are double-stranded DNA viruses whose genetic material is protected by a protein capsid of icosahedric geometry, with no external covering, an icosahedric capsid whose vertices originate from thin extensions called fibres, so that the structure of the complete viral particles is similar to the one presented in Fig. 1. Its viral genome contains approximately 36 Kb, flanked by two ITR (*inverted terminal repeats)* of between 100 and 140 bp, each of which constitutes one of the ends of the adenoviral genome and whose principal function is to initiate replication of the virus. The packaging signal (Ψ) is located very close to one of these ITR; this addresses the encapsidation of the viral genome in the capsid thanks to the interaction with different adenoviral proteins which are characterized by containing a variable number of type A repetitions (TTTGN8CG), depending on the serotype[31]. The end of the adenovirus genome closest to the localization of the packaging signal is normally considered as the 5' extreme, since it is also the end that is closest to the E1 region which contains the genes which are first expressed. The genome is divided into early genes (E1-E4), which are transcribed in the first phases of the cycle, just before replication of the viral DNA; and late genes (L1-L5), transcribed in a later phase (protein production and encapsidation).

More than 100 adenovirus serotypes have been identified, some of which are capable of infecting mammals and, others, birds. Adenoviruses capable of infecting mammals include those capable of affecting human beings, organisms whose principal target is found in the respiratory tract where they typically cause colds or even pharyngitis. Different adenoviruses can also cause different types of conjuntivitis and, less frequently, cystitis and gastroenteritis. Adenoviruses are capable of infecting other different organisms, although normally the infections caused by them are asymptomatic. Currently over 50 human serotypes and various adenovirus serotypes for other animals such as dogs, cows, etc. have been described.

Of the different serotypes capable of infecting human beings, the one most used in gene therapy testing is serotype 5 (Ad5), associated principally with mild respiratory infections. Its genomic sequence can be found in the GenBank database with the code AC_000008 (http://www.ncbi.nlm.nih.gov/entrez/viewer.fcgi?db=nucleotide&val=56160529). In this sequence the ITR correspond to the first 103 nucleotides and to the final 103 nucleotides of the virus. The packaging signal extends from nucleotide 200 to 400, accounting for nucleotides beginning with the first nucleotide found in the end marked as 5', and contains 7 type A repetitions (TTTGN8CG). A drawing of the genome map for serotype 5 adenovirus can be seen in the top part of Fig. 2.

In general when adenoviruses infect human cells in cultures, particular when dealing with serotype 2 and 5 adenoviruses, these lead to productive infections where the viral genome is transcribed in the nucleus, the mRNA is translated in the cytoplasm and the virions (complete viral particles) are packaged in the nucleus. Cells in which the adenovirus leads to a productive infection and causes lysis, releasing formed viral particles, are known as permissive cells. A receptor is required in the cellular membrane of the cell for the adenovirus particles to penetrate the cell; this is better known and characterized as the CAR protein (*Coxsackie B virus and adenovirus receptor),* a protein that expresses itself at variable levels in different tissues and in different phases of development; this is the principal protein responsible for susceptibility to infection by adenoviruses, although the presence of the so-called integrines, proteins that faciliate the entry of the viral particle through receptor-mediated endocytosis thanks to its interaction with penton proteins, is also important. The best characterized of these integrins are $\alpha_V\beta_5$ and $\alpha_V\beta_3$. The different serotypes can use different receptors and integrins. The viral particles interact with the receptor principally through the so called "fibres", which are thin projections that begin in the vertices of the icosahedron that is formed by the proteins making up the adenovirus capsid, in which vertices the so called "pentons" are found, which are pentameric complexes that constitute the base of the fibre protein. It is therefore the fibre what principally defines the types of cells that each adenovirus is capable of infecting, which will be those that possess the appropriate receptors for the adenovirus fibre to interact with them. Fig. 3 demonstrates how the internalization of the viral particles is produced in the cell and their penetration into the nucleus, where the genomic DNA is released.

Wild-type adenoviruses require 28-48 (sic) from the time they enter the cell in order to complete the multiplication and packaging processes and to cause cellular lysis. Most adenoviruses, including the human serotype 5 adenovirus, cause lysis of the host cell approximately 36 hours after entering the same. In these cases, the intranuclear virion begins to form after 8 hours, and from $10^4$-$10^5$ particles can be produced per cell.

1st Generation Adenoviruses

Illnesses caused by Ad, even when associated with well described pathologies, generally are relatively unimportant. To prevent the replication and propagation of these vectors and their adverse effects for possible use in gene therapy, vectors must be generated that are deficient in replication. First generation vectors are deficient in the E1 region, which is the first region of the Ad to transcribe and lead to the different stages of replication. Since adenoviruses cannot efficiently package genomes that are larger than approximately 105% of the size of the wild type genome, the vector generated from the serotype 5 adenovirus allows a maximum capacity of 5.1 Kb (Kilobases) for introducing of a therapeutic gene.

The adenoviruses lacking the E1 region cannot replicate their genome. Therefore if their multiplication is desired, the permissive cells used must be able to complement the function of the E1 region. That is the case, among others, of the HEK293 cell line, commonly referred to as simply 293, a cell line from an embryonic human kidney frequently used to amplify adenoviruses, which gives a stable expression of the proteins corresponding to genes E1a and E1b and allows the replication of the first generation adenoviruses. Other examples of permissive cells for serotyp 5-derived first generation helper adenoviruses are PERC6[17] (cell line deriving from human retinoblats) and N52E6[20] (cell line deriving from human amniocytes).

First generation adenoviruses were later created in which the E3 region was also eliminated, to increase their biosecurity. As a result of the elimination of the E3 gene, their capacity to incorporate genetic material increased to 8.2 Kb. The E3 region plays an important role in avoiding host defense mechanisms, and is not fundamental for replication[4].

1 st generation vectors, although they cannot replicate by themselves, generate a significant *in vivo*[5] immune response due to the residual expression of viral proteins[6], which activates the cell immune response mediated by cytotoxic T lymphocytes which in turn eliminate the transduced cells, and thereby transgene expression[7]. The creation of 2nd generation vectors in which the E1 and E3 regions have been eliminated as well as zones of regions E2 and E4, did not completely resolve this problem.

Gutless adenoviruses or helper-dependent adenoviruses

Gutless Ad, also known as *helper*-dependent (*helper-dependent adenoviruses:* HD-Ad), or high capacity (*high capacity adenoviruses:* HC-Ad), were created to prevent the problem of Ad-created cellular immune response. The term *gutless* refers to the fact that all encoding regions of the Ad have been eliminated; they are helper-dependent, because they depend on a helper adenovirus to be able to produce; and they are of high capacity because they allow insertions of up to 36 Kb. Residual protein expression is eliminated by deleting the entire coding viral region, thus preventing any immune reaction against the viral proteins and increasing transgene persistence. Gutless Ad maintain only the 5' and 3' ITRs and the packaging signal ($\Psi$), which is essential for final assembly of the virion.

The gutless Ad have been administered *in vivo* to different tissues such as liver[24,25], muscle[26], central nervous system[27], retina[28], lung[29] and uterus[30] in different animal models such as mice, rat, dogs and non-human primates (baboons). Since the Ad, administered by systemic route, exhibits high transduction effciency by liver, in most studies with gutless Ad liver is selected as the target organ. This organ is also very attractive for gene therapy due to the fenestrated structure of its endothelium which has openings that allow the Ad to come into contact with the parenchyma. The central nervous system (CNS) is also very attractive for studies with gutless Ad, since the neurons do not replicate and, for this reason, transgene expression could be maintained during long periods of time if neurons are transduced *in vivo*, including throughout the entire life of the animal. Finally, muscle is also one of the organs most used in studies of gutless Ad, since it comprises 40% of the total body mass, is highly vascularised and skeletal fibres can be transduced *in vivo*. Myofibres present a slow cellular duplication, which guarantees a stable expression over time.

However, even though they exhibit a high number of advantages, their use in clinical tests is still questionable due firstly to the elevated contamination which is normally caused by the so called helper Ad, and secondly because an efficient system has not yet been developed for their large scale production in a bioreactor.

Since gutless Ad have no viral encoding region, in order to achieve its multiplication this Ad must be transduced with an Ad that provides the proteins necessary for replication and packaging *in trans*: this last type of adenovirus is known as a helper adenovirus. In addition a cell line must be used that can restrict the growth of the helper Ad with respect to the gutless Ad. The later separation of these two vectors is essential in order to obtain a pure vector with no contamination from the helper Ad. Strategies used to separate both vectors have evolved over the past 10 years, from physical separation by centrifugation[8], the use of specific recombinases that eliminate the helper Ad packaging signal, up to the specific mutation of the helper Ad packaging signal to reduce its encapsidation[10-12].

Historically the first production system consisted of the physical separation by centrifugation through a CsCl gradient, of an Ad with large deletions in its coding region with respect to a wild type Ad. Growth of the wild type Ad allowed the propagation of both Ad. However high levels of contamination from wild type Ad were detected after purification, as well as various recombinations between them[8].

The system that contributed to a major advance in reducing helper Ad contamination consisted of the specific deletion of that adenovirus packaging signal by specific recombinases. The helper Ad signal $\Psi$ was flanked by loxP sequences which were specifically recognized by the Cre recombinase, which was expressed by a cell line, 293Cre[9]. This cell line was created specifically for this purpose from one of the cell lines customarily used for adenovirus multiplication, i.e. the line from the embryonic human kidney known as HEK293 or simply 293. As demonstrated in Fig. 4, when both helper and gutless Ad were co-transduced in this cell line, the Cre recombinase excised the region located between the two loxP sequences and, with this, the $\Psi$ signal of the helper Ad and, consequently, the genome could not be packaged. However it could provide the proteins needed, *in trans*, for growth of the gutless Ad. Mean helper Ad contamination varied between 0.1-10%; consequently this contamination must be reduced even further for possible clinical use.

FLPe recombinase, derived from yeast, which specifically recognizes frt signals, was used for the same purpose. Cell lines 293FLPe and 293CreFLPe were generated together with helper Ad whose $\Psi$ signal was flanked by frt sequences. However neither the use of the FLPe cell line nor the double cell line 293CreFLPe presented an advantage in increasing the elimination of the $\Psi$ signal.

Alternative viruses are also being used which provide the proteins necessary for Ad packaging *in trans*. The herpes simplex virus-1[13] and baculoviruses[14] have been used in this way. Both systems present a low production efficiency[15] and generate high percentages of replication-competent adenovirus particles (RCA)[14], and therefore they must be improved in order to proceed with large scale production.

## The ϕC31-attB/attP System

One of the limitations of the Cre-loxP and FLPe-frt systems is that they present bi-directional activity, that is in conditions of high enzyme concentration, they can act in both reaction directions, making it difficult to reduce helper Ad contamination to levels below 0.1 %. Cre recombinase is also toxic to the cell at the high levels[36] necessary to completely excise the $\Psi$ sequence. The group of the inventors had therefore previously proposed the use of methods involving the intervention of one-directional recombinases as a method that supposedly presented an advantage, firstly to reduce helper Ad contamination[20], and secondly because cell toxicity processes associated with their over-expression have not been described. More specifically, methods were proposed that used ϕC31 recombinase, derived from the ϕC31 bacteriophage, a virus that infects bacteria of the *Streptomyces* genus and which is capable of integrating into its genome. The ϕC31 recombinase is functional both in prokaryotic cells as well as in mammal and human cells[21]. The bacteriophage genome integrates into the bacterial genome thanks to the fact that the recombinase specifically recognizes sequences known as attB (present in the bacterial genome) and attP (present in the bacteriophage genome), which are different in terms of sequence and number of nucleotides. Upon recombination between the attB and the attP sequences, the ϕC31 recombinase generates 2 new sequences called attR and attL. These new sequences are not recognized in mammal cells by the ϕC31 recombinase; as a result the system can act only in one direction. In addition the ϕC31 recombinase shows levels that are similar, in terms of activity, to the Cre recombinase in 293 cells[22], as demonstrated in cell lines that expressed said ϕC31 recombinase, such as the 293ϕC31 line[22], which cell lines, like the lines which expressed an additional recombinase such as the 293CreϕC31 line, had been planned to be used to produce gutless adenoviruses. The system initially proposed involved the use of a method to produce gutless Ad with helper Ad that carried their packaging signal flanked by an attB signal on one side and an attP signal on the other, a system that was expected to achieve contamination levels inferior to those customarily found when applying production methods based on bidirectional recombinases. Nevertheless as occurs with the rest of the recombinase-based systems, application of that production method implied both the creation and use of cell lines capable of expressing the corresponding recombinase, such as lines 293ϕC31 and 203CreϕC31 mentioned above, as well as construction of adenoviruses, intended to be used as helper adenoviruses, comprising both an attB as well as an attP sequence, each one at a different end of the packaging signal. Just as the system was designed, the only requirement for the adenovirus was having the Ψ sequence flanked by the attB and the attP sequences to allow the recombinase-mediated excision of said Ψ sequence, but, according to scientific literature available, which indicates that the side where each of the sequences recognized by the recombinase is flanking the sequence planned for excision is independent, the selection of the region next to the specific packaging signal where each of the attB and attP sequences are to be placed did not appear to be relevant.

The future of gutless Ad in gene therapy depends on the development of efficient systems that will allow large scale production in bioreactors as well as isolation with low levels of helper Ad contamination. A system that would allow the multiplication of gutless Ad and their purification with helper Ad contamination levels inferior to those achieved with the recombinase-based systems customarily used until now, without necessarily requiring the use of cells capable of expressing a determined recombinase and avoiding the toxicity problems associated with their over-expression, would represent an important step in facilitating production and improving safety. The method of the invention exhibits both characteristics.

## DESCRIPTION OF THE INVENTION

Systems based on flanking the Ψ sequence with specific recombinase-recognized sequences required cotransduction of the gutless adenovirus and the helper adenovirus in a cell line that could express said recombinase and which, by acting on sequences recognized by it, located flanking the Ψ sequence of the helper adenovirus, would excise this latter sequence, preventing the packaging of the helper adenovirus. System efficiency therefore depended on the efficiency of the recombination process catalyzed by the recombinase.

Surprisingly, it has now been found that the simple presence of an attB sequence recognized by the recombinase of the *Streptomyces* ϕC31 bacteriophage flanking the 5' end of the Ψ sequence of the helper adenovirus gives rise to a sensible delay in the packaging process of the helper adenovirus in comparison with that of the control adenovirus. Based on this, the invention provides a method for the production of gutless adenoviruses that allows for obtaining the latter with a level of helper adenovirus contamination that is lower than the level obtained with the use of methods known until now, and which do not necessarily require the use of specific cell lines that express a determined recombinase, but rather which is based on careful selection of the time of recovering the gutless adenovirus from the culture.

The invention refers to a method for the production of gutless adenoviruses essentially free of helper adenovirus comprising the steps of:

a) cotransducing the gutless adenovirus genome together with a helper adenovirus genome comprising a ϕC31 attB sequence located 5' to the packaging signal Ψ, in a permissive cell line in which the expression of helper adenovirus proteins and the replication of the genomes of both adenoviruses is feasible;
b) cultivating the cells under conditions where the expression of helper adenovirus proteins and the replication of both adenovirus genomes is possible;
c) recovering the gutless adenovirus from the cell culture after a time period from cotransfection enough for complete viral particles of gutless adenovirus to be formed and less than that needed for the helper adenovirus to complete its viral cycle.

This method allows for obtaining gutless adenoviruses with contamination levels below those obtained using current methods. It is for this reason that we say that a gutless adenovirus is obtained that is essentially free of helper adenovirus. As used in the invention, "gutless adenovirus essentially free of helper adenovirus" means that the level of helper adenovirus present in the final preparation of gutless adenovirus is at least less than 0.01%. Given the characteristics of this method of the invention, we can expect the level of contamination by helper adenovirus obtained using this method to be less than 0.001 %.

Thus, the term transduction includes the processes of penetration of DNA sequences that are not associated with adenoviral proteins forming complete particles as well as those processes wherein the DNA sequence was in the exterior of the cell, prior to its penetration, associated with adenoviral proteins forming complete particles. As used in the present application, the term "transduction" implies the entry of a DNA sequence into a cell, independently of whether the sequence is associated or not with viral proteins forming a complete viral particle, and also independently of whether the entry mechanism is simply the natural entry mechanism of the infectious particles of adenoviruses in the cells (the endocytosis mediated by a receptor described above), or whether the entry mechanism is another different mechanism, such as those involving the intervention of agents with a negative charge that facilitate the entry of DNA molecules into the cell. Accordingly, "transducing an adenovirus genome", as used in the present application, implies the entry into the cell of a DNA sequence comprising at least one adenovirus packaging signal Ψ located between two adenovirus ITR located in such a way that the bicatenary sequence of nucleotides comprised between both ITR complies with the conditions specified below as conditions allowing the double-stranded sequence to be packaged in an adenovirus capsid and giving rise to complete viral particles. In this way, the expression "transducing an adenovirus genome" refers to both the entry of the corresponding sequence of DNA associated with viral proteins forming a complete adenovirus particle as well as its entry without forming part of a complete adenovirus particle, by any mechanism different from that of endocytosis mediated by a receptor.

As used in the present application, the term "adenovirus genome" refers to a double-stranded DNA sequence that, in the presence of appropiate proteins, can be packaged, resulting in a complete adenovirus particle. For this packaging to occur, the sequence must comply with some conditions, which can be summarized as follows:

- exhibit separate adenovirus ITR, one at each of its end points;
- comprise a packaging signal Ψ between both ITRs, located in such a way that the distance between the 5' end of the packaging signal Ψ and the 3' end of the ITR closest to it does not exceed the distance that would prevent packaging of the natural adenovirus, a distance that is 200 base pairs in the case of the human serotype 5 adenovirus and which is assumed, by analogy, to be approximately equal in the case of other serotypes, since it has been seen that the introduction of sequences between the ITR and the packaging signal in the sequence that naturally separates them decreases the packaging capacity of the adenoviral genome[35], causing a reduction in the total number of adenovirus particles obtained, even though there is no significant change in the time necessary for their packaging;
- the distance between the ends of both ITR should not be greater than 105% of the size of the adenovirus genome present in nature to which the proteins which will form the capsid belong.

Fig. 2 represents sequences that meet these conditions: the first of them corresponds to the genome of a natural adenovirus, specifically a human serotype 5 adenovirus; followed by sequences corresponding to so called first and second generation helper adenoviruses, and finally a sequence in the lower section, labeled as "*Gutless*", that continues to comply with the conditions set forth in order to be considered an adenovirus genome. In the gutless sequence, the sequence located between the packaging signal Ψ and the ITR most distant from it generally does not derive from the genome of natural adenoviruses and usually comprises a coding sequence of interest for gene therapy in humans. In order for the sequence corresponding to a gutless adenovirus genome to be packaged, resulting in complete adenovirus particles similar to those represented in Fig. 1, it is necessary the presence of the proteins that form the capsid. In the method of the invention, the packaging of the gutless adenovirus occurs in a cell, where the proteins that form part of the complete adenovirus particle generally result from the expression of the genome of a helper adenovirus present in that same cell, although it could also happen that some of them would have been produced by the expression of one or more genes stably transfected in said cell. Furthermore, for the multiplication of the gutless adenovirus to occur, there must have been replication of its genome which, in turn, requires the presence of enzymes that can also be supplied by the helper adenovirus or, if the helper adenovirus lacks the corresponding genes, they can be complemented by the cell in which multiplication of the gutless adenovirus occurs. To assure the replication and packaging of the gutless adenovirus, it is advisable that its ITRs and Ψ packaging signal should come from the same serotype as those present in the helper adenovirus used, which can be replicated and packaged in the cells used.

Therefore, the application of the method of the invention involves the simultaneous presence in the same cell of the DNA sequence corresponding to the genome of a gutless adenovirus and that of an adenovirus that can act as helper to make it possible for both the gutless adenovirus as well as the helper adenovirus genome to replicate, and also to provide the proteins necessary for the gutless adenovirus genome to be packaged and, thus, to give rise to complete particles of adenovirus. As used in the present application, "cotransducing the genome of the gutless adenovirus together with the genome of a helper adenovirus" refers to compliance with this requirement, to make possible the simultaneous presence of the genome of a gutless adenovirus and the genome of a helper adenovirus in a single cell. As noted before, when they enter the cell, the DNA molecules corresponding to the helper adenovirus genome as well as those corresponding to the gutless adenovirus genome may or may not form part of complete adenovirus particles. All the possible combinations related to the ways the gutless adenovirus genome and the helper adenovirus genome penetrate the cell are all of them embodiments of the method of the invention that are included within its scope, that is: when the helper adenovirus genome penetrates the cell as a part of a complete adenovirus particle, then the entry of gutless adenovirus genome may or may not occur being part of a complete particle of adenovirus, because the introduction into the cell may occur in a different manner such as for example, being mediated by some agent that facilitates penetration of DNA molecules into the cell. On the other hand, when the helper adenovirus genome penetrates the cell without being associated to adenovirus proteins to form a complete adenovirus particle, but rather in any another way, then it is also equally possible that the gutless adenovirus genome will penetrate into the cell without forming part of a complete adenovirus particle, through some mechanism different from that of the endocytosis mediated by an adenovirus receptor, or it may also occur that the sequence corresponding to the gutless adenovirus genome is indeed associated to adenoviral proteins forming a complete adenovirus particle, thanks to which its entry into the cell could be via receptor-mediated endocytosis.

Entry of adenovirus genomes into the cell will preferably occur being associated to adenoviral proteins forming a complete adenovirus particle, via the mechanism of receptor-mediated endocytosis described above. When the helper adenovirus genome and/or the gutless adenovirus genome is transduced without the corresponding DNA molecule being part of a complete adenovirus particle, then, for the entry into the cell to occur, it is convenient to resort to any other classic system that facilitates the penetration of DNA molecules into a cell, such as adding to the cell culture: calcium phosphate, lipofectamine or cationic polymers such as PEI (polyethylenemine)[34] or any other system known to the skilled in the art, whose specific use conditions can be easily determined by any of them.

Choice of the recovery time of the gutless adenovirus is important in order to achieve high titres of the same with the possible minimal contamination by helper adenovirus. It is therefore important to know both the moment of finalization of its cycle as well as the time of finalization of the helper adenovirus which was transduced with it in order to perform the method of the invention as appropiately as possible. For all these reasons, included within the method of the invention are those methods that comprise a previous step wherein the duration of the cycle of the helper adenovirus and the duration of the cycle of the gutless adenovirus cycle specifically used are determined in the cell line and the growing conditions to be used to proceed with multiplication of the gutless adenovirus, as well as the moment when packaging of each of said adenoviruses can first be detected and the evolution of the number of viral particles produced per cell over time are also determined, so that step c) cited above can be carried out as defined, that is, beginning the process of recovering the gutless adenovirus prior to the finalization of the viral cycle of the helper adenovirus. Duration of the viral cycle of the gutless adenovirus and the helper adenovirus can be determined in an analogous form to the process described in Example 4 which appears later in the present application, or by any other method known by the skilled in the art.

As used in the present invention, an adenovirus is considered to have completed its viral cycle when lysis is caused in the cell in which it is located, thus releasing the completely packaged viral particles, which are then free to penetrate a new cell. The moment of recovery of the gutless adenovirus from the cell culture in which it is multiplying can be selected so that this is either prior to or after the finalization of its viral cycle and release from the cell. As commented above, the formation of viral particles can first be detected several hours after the adenovirus genome penetrates the cell. The period of time for the adenovirus most customarily used in tests carried out for future use in gene therapy, which is human serotype 5, is about 8 hours. From that moment on, it could be already possible to think of recovering particles from the culture, purifying them directly from the cells themselves. The amount recovered under those conditions would logically be very small; so it is preferable that they be left for more time, to allow a larger number of adenovirus particles to form. As time passes, the adenovirus particles will continue to accumulate in the cell nucleus, until the adenovirus cycle is completed, the cell lyses and the particles pass to the culture medium. The time selected to recover the particles of gutless adenovirus therefore determines if these ones are recovered (that is, purified and concentrated) from the cells themselves or from the culture medium. Both those in which the moment of recovery of the gutless adenovirus is prior to finalization of its cellular cycle and its release from the cell by lysis as well as those when recovery is after cell lysis are embodiments of the method of the invention.

If recovery is made from the cells, before lysis occurs, then the moment of recovery selected will preferably be very close to the finalization of the viral cycle, so that there is a very high number of particles of gutless adenovirus accumulated in the cell and the efficiency of adenovirus recovery is much higher. To proceed with the recovery of gutless adenovirus from the cells, it will be necessary to separate the cells from the culture medium using any method known by the skilled in the art, to disrupt the cells in inert solution and to purify the particles of adenovirus by using known methods such as ultracentrifugation in CsCl or FPLC (fast liquid protein chromatography). Recovery from the cells, prior to lysis, can be the selected method when working with small culture volumes.

When working on a large scale, that is when the range of magnitude of millilitres or centilitres of cultures has been surpassed so that cultures are run in large volume reactors (the most common of which are currently of 2 litres of capacity or above, although there are also 0.5 litre reactors), recovery of the gutless adenovirus from the culture medium may be more appropriate. As such the time of recovery of said adenoviruses is chosen in such a way that their viral cycle has finalized, lysis has occurred in the cells where they have multiplied and packaged, and they have passed to the cell culture medium. Again it is convenient that the time for proceeding with recovery be near, although after, the finalization of the viral cycle of the gutless adenovirus and, in any event, prior to the finalization of the helper adenovirus viral cycle. In this way the presence in the culture medium of particles of helper adenovirus produced in cells in which there was no cotransduction is avoided to the extent possible, the helper adenovirus is the only one that has multiplied itself and the cell lysis is caused by finalization of the viral cycle of the helper adenovirus, whose cycle finalizes after the finalization of the viral cycle of the gutless adenovirus. Therefore, it is preferable that the time of recovery of the gutless adenovirus from the culture medium is later than, but close to the moment of finalization of the viral cycle of the gutless adenovirus. When this embodiment of the invention is selected, particles of gutless adenovirus can be recovered from the culture medium using purification techniques known to the skilled in the art, such as for example FPLC which allows the purification of large amounts of various biomolecules and particles and which makes working with large volumes feasible.

Whether the gutless adenovirus is recovered from the cells or from the culture medium, it is important to know the time of finalization of the viral cycles of each of the adenoviruses, gutless and helper, in order to select the time to proceed with said recovery. The specific characteristics of the helper adenoviruses being used will be determinant for this.

As previously noted, when ϕC31 bacteriophage infects *Streptomyces,* it is capable of integrating into the bacterial genome. This requires the expression of a protein, the bacteriophage recombinase, herein referred to as ϕC31 recombinase, which recognizes and binds two sequences, one in the bacterial genome (referred to as attB) and the other in the bacteriphage genome (attP). The recombinase makes a cut over the attB sequence, inserting the bacteriophage genome between the two resulting endpoints. The complete attB sequence corresponding to ϕC31 bacteriophage has a length of approximately 500 bp[32], although recent studies have shown a 34 pb-minimal region[21] comprised of a nucleus with TTG nucleotides and a region rich in cytosines (C) and guanines (G) flanking the nucleus, which is GTGCCAGGGCGTGCCCTTGGGCTCCCCGGGCGCG (SEO ID NO:1)

However, in the method of the invention, the helper adenovirus used to make the multiplication of the gutless adenovirus possible presents an attB sequence that is somewhat longer, 53 bp, containing the 34 bp-minimal sequence and additional nucleotides that flank this 34 bp-sequence in the complete attB sequence present in nature, thus achieving a sequence that would guarantee a greater efficacy if the action of the recombinase is wished. This sequence is as follows:

```
5′CCGCGGTGCGGGTGCCAGGGCGTGCCCTTGGGCTCCCCGGGCGCGTACTCCAC 3′ (SEQ ID NO:2)
5′     GTGCCAGGGCGTGCCCTTGGGCTCCCCGGGCGCG               3′ (SEQ ID NO:1)
```

As used in the present application, the attB sequence of ϕC31 bacteriophage is considered to be any sequence comprising said 53 bp represented by SEQ ID NO:2. The expressions "attB sequence corresponding to ϕC31 bacteriophage", "ϕC31 attB sequence", "attB sequence of ϕC31 recombinase", or simply "attB sequence" also refer to any sequence which comprises that minimal region of 53 bp.

Also comprised within the scope of the invention are the embodiments that use as helper adenoviruses those that exhibit the attB sequence corresponding to SEQ ID NO:2, as well as those exhibiting the larger sequences comprising SEQ ID NO:2 as attB sequence.

The method of the invention requires the DNA sequence corresponding to a gutless adenovirus being cotransduced together with the DNA sequence corresponding to a helper adenovirus presenting an attB sequence of ϕC31 recombinase located between the packaging signal Ψ and the end of the adenovirus genome that is closest to that Ψ sequence. The structure of the helper adenovirus DNA can correspond to the structure represented in the upper part of Fig. 2, immediately below the scale, that is, comprise the complete genome of an adenovirus, or it can also correspond to any of the structures represented below (except that corresponding to the gutless adenovirus), deriving from the deletion of part of the gene sequences, forms that correspond to first and second generation adenoviruses. The use of adenoviruses that lack the E1 region is preferred, to prevent the possibility of the minimal amount of helper adenovirus that could be present in the final preparation of gutless adenovirus from being competent for replication.

It is possible that both the ITRs as well as the packaging sequences and all the gene sequences present in the adenovirus used as helper come from the adenovirus genome of the same serotype; however it is also possible to use chimera adenoviruses, formed from the combination of elements from different serotypes, which serotypes can infect different species in nature. Sequences that come from adenovirus genomes present in the genome of the helper adenovirus used in the method of the invention preferably derive, either all of them or most of them, from human adenovirus serotype 5, and especially the ITRs should correspond in their sequence with the 103 first nucleotides and the 103 last nucleotides of the sequence of GenBank database accession number AC_000008, the packaging signal should correspond with the sequence comprised between nucleotide 200 and nucleotide 400 fo the mentioned GenBank sequence, with nucleotides counted from the one located at the 5' end of the GenBank sequence, and the majority or all of the coding sequences present in the helper adenovirus and the promoters driven their expression should also come from serotype 5 adenovirus. Nevertheless the use of helper adenoviruses from other serotypes is also compatible with the method of the invention, including non-human serotypes such as for example canine adenovirus CAV-2 which may be the only serotype from which all sequences from adenovirus genomes come, or as mentioned before, can be the source for just one part of the elements conforming the chimera helper adenovirus genome, thus allowing that other elements from the helper adenovirus genome come from one or more different serotypes.

Among the possibilities offered by the use of helper adenovirus chimeras are those where, for example, the coding sequence corresponding to the fibre protein and/or the sequence coding for the protein that forms the penton (the part of the capsid that interacts with the integrins) proceed from a different adenovirus serotype than the rest of the sequences. In that case, the sequences that do not correspond to the fibre protein and/or to the protein that forms the penton, come preferably from an adenovirus serotype for which cell lines are available that are capable of complementing the function corresponding to the E1 region of the adenovirus, which will allow working with helper adenoviruses whose genome lacks that region. Currently, cell lines are available that are capable of complementing the function of the E1 region of human serotype 5 and canine CAV-2, and these are therefore the serotypes preferred for construction of helper adenovirus chimeras.

The cells used for multiplication of the gutless adenovirus will be determined mainly by the serotype of the helper adenovirus used. They can be any permissive cells, that is, cells in which the adenovirus leads to a productive infection and causes their lysis, thereby releasing the viral particles formed. To do this it is necessary to produce both the replication of both the gutless and the helper adenovirus genomes, and also to synthesize the proteins necessary to complete the process, among these the proteins that form part of the viral particles and those that make their packaging possible, It is therefore necessary for all the proteins required to complete these processes to be present in the cell. Except for the proteins synthesized from the E3 region, no other adenovirus protein is essential for replication and packaging of the gutless. Thus, all the genes deleted from the genome of the adenovirus used as the helper, with the possible exception of those from E3 region, must be complemented by the cell line used for multiplication of the gutless adenovirus. Thus for example, if the helper adenoviruses used have had their E1 region deleted, then cell lines capable of complementing the function corresponding to the proteins synthetised from said E1 region must be used. If helper adenoviruses are used that derive from human serotype 5 adenoviruses and which lack E1 region, they would be permissive cells in which genomes from the gutless and the helper adenoviruses could be replicated and packaged to give rise to viral particles the cells corresponding to lines such as HEK293, PERC6 or N52E6, or any other that complies with the conditions to be permissive and to complement the functions that are absent from the helper adenovirus. As noted before, at this time cell lines that provide a stable expression of the proteins corresponding to E1 region of each serotype are not available, except in the case of Ad5 and CAV-2. For this reason chimera adenoviruses are frequently used which derive mostly from Ad5 but whose fibre protein is from another serotype.

When the penetration of the DNA sequences of the helper and/or gutless adenoviruses occurs in the form of complete adenovirus particles, via the receptor-mediated endocytosis mechanism, then the surface of the cell membrane must also possess receptors adequate to interact with the fibres that extend beyond the capsids of the helper adenovirus and/or the gutless adenovirus, and integrins that favour internalization of the viral particle. In the case of helper adenoviruses deriving from Ad5, cell lines such as the before mentioned HEK293, PERC6 or N52E6 are valid, or any other permissive cell in which the adenovirus particles can penetrate and where the replication of helper and gutless adenovirus genomes, the expression of adenovirals and the packaging of new particles can occur. However contrary to the methods described in the state of the art, it is not necessary for the cells in which the gutless adenoviruses are cotransduced and where they multiply themselves to express any type of exogenous recombinase, and so, the method of the invention allows the amplification of the repertoire of cells useful for the production of gutless adenovirus.

On the other hand, although the method of the invention is based on using the difference between the time of packaging of the gutless adenovirus and that of the helper adenovirus when the latter contains an attB sequence located between the packaging signal Ψ and the ITR sequence located at the end of the adenovirus genome closest to said signal Ψ, it is compatible with the combination with classic systems based on the use of recombinases to contribute a lower degree of contamination by helper adenovirus in the gutless adenovirus obtained, provided that the helper adenovirus used comprises an attB sequence in the localization indicated and there is still a delay in the packaging moment of the helper adenovirus with regard to the gutless adenovirus which could be advantageous to determine the time to proceed to isolation of the gutless adenovirus. Therefore, embodiments wherein the helper adenovirus used, additionally to exhibit the attB sequence, has its Ψ signal flanked by sequences that allow recognition by recombinases and wherein the cells in which the gutless adenoviruses and the helper adenovirus are cotransduced express the recombinase that recognizes said flanking sequences, are also embodiments of the method the invention, encompassed in its scope. One of the possibilities is to use, in the method of the invention, adenoviruses that comprise an attP sequence of ϕC31 bacteriophage flanking the packaging signal Ψ at the end opposite to the that where the attB sequence is found, and to cotransduce the helper adenovirus and the gutless adenovirus in a cell line that expresses ϕC31 recombinase, such as cell line 293ϕC31; in this case, it is preferable that the attB sequence is separated from its corresponding attP sequence by 1.5-2 kb, to try to favour the activity of the ϕC31 recombinase which, like all recombinases, varies its activity based on the number of base pairs found among the specific sequences recognized by it. The helper adenovirus comprising an attB sequence and optionally, an attP sequence can have its Ψ sequence also flanked by sequences recognizable by other recombinases, such as for example loxP sequences or frt sequences. In this case, the method of the invention could be carried out by the cotransduction of the helper adenovirus and the gutless adenovirus in cell lines that express, respectively, Cre recombinase (such as for example cell line 293Cre) or FLP recombinase (such as for example cell line 293FLPe). It is also possible to use cell lines that express more than one recombinase functional in eukaryotic cells, such as line 293CreϕC31 (which expresses the Cre and ϕC31 recombinases and could be a cell to select when using with helper adenoviruses having an attB sequence of ϕC31 and a loxP sequence located 5' to the packaging signal Ψ, as well as an attP sequence of ϕC31 and another loxP sequence located 3' to the packaging signal Ψ.)

Cell culture conditions are not determinant for application of the method of the invention, provided that conditions are used that are appropriate for the cells used. In general terms, one can considered as adequate standard culture conditions, at 37°C and an atmosphere of 5% $CO_2$, using DMEM as a culture medium (Dulbecco modified Eagle medium,) + FBS (Fetal Bovine Serum), the latter in a percentage that can vary from 1% to 10% without affecting the development of the viral cycle.

The examples given further in this document describe the construction of various helper adenoviruses that present the characteristic mentioned for being valid for the application of the method of the invention in combination with systems that use recombinases: Ad5FC31 (which possesses an attB sequence flanking the Ψ sequence inserted between this and the ITR closest to it and an attP sequence in the region opposite to the Ψ sequence, at 2 kb from the attB sequence), Ad5/loxP.FC31 (which possesses an attB sequence flanking the Ψ sequence inserted between this and the ITR closest to it and an attP sequence in the opposite region to the Ψ sequence, at 2 kb of the attB sequence, as well as a loxP sequence between the attB sequence and the Ψ sequence), Ad5/attB.Cre (which possesses in an attB sequence flanking the Ψ sequence inserted between this and the ITR closest to it, a loxP sequence between the attB sequence and the Ψ sequence and another loxP sequence in the region opposite to the Ψ sequence, at 2 kb from the loxP sequence) and Ad5/FC31.Cre (which possesses en an attB sequence flanking the Ψ sequence inserted between this and the ITR closest to it and an attP sequence in the opposite region to the Ψ sequence, at approximately 2 kb from the attB sequence, and two loxP sequences, one located between the attB sequence and the Ψ sequence and the other located between the attP sequence and the Ψ) (see Fig. 5). Any of them shows a significant cell cycle delay of between 14 and 20 hours with respect to the control adenoviruses, when amplified in 293 cells lacking recombinases of exogenous origin, without an effect on the replication processes of the viral genome, the processes of expression and production of viral proteins and the maturation processes of the viral capsid, since it is the packaging process of its genome that is altered by being much slower than in the control adenoviruses. All of them are susceptible to possible use as helper adenoviruses in the method of the invention, since they comply with one of the most relevant conditions to be able to act in a gutless adenovirus production system, which is that the helper adenovirus used can supply the viral proteins for its replication and that this is done during the viral cycle of the gutless adenovirus. The protein production analyses showed in the examples indicate that the adenoviruses indicated are adequate for use as helpers in the method of the invention, and they are not retained in nuclear regions with low or null protein expression, despite the delay in their packaging moment. Also significant, the delay in the packaging moment is not recovered in the presence of normal viral proteins supplied in trans by control adenoviruses. The effect due to the presence of the attB sequence is the appearance of a delay in the moment when packaging is possible, but not a loss of the capacity of the adenovirus genome containing the sequence for packaging itself provided that enough time is allowed to pass; the tests described below in the examples show that the adenoviruses with attB sequences can be produced at levels similar to the controls lacking an attB sequence if sufficient time is allowed to pass to complete their viral cycle. This effect of delaying the packaging time is observed in all adenoviruses in whose genome an attB sequence was inserted between the packaging signal Ψ and the ITR sequence located at the adenovirus genome end closest to said sequence Ψ, but is not observed in adenoviruses lacking attB sequences even though an attP sequence was present in the same at a distance from 1.5 to 2 kb from the attB sequence (as occurs in the adenovirus used as control, Ad5attP, in which no delay in packaging time was observed with respect to the 36 hours cycle considered normal in 293 cells). As shown by the tests performed with adenovirus Ad5attBCre, the presence of an attP sequence at an appropriate distance from the attB sequence seems not to be necessary either to produce the effect of delaying the packaging time.

The helper adenoviruses designed and constructed until now by introducing into their genome recombinase-recognizable sequences flanking the packaging signal would always require the presence of sequence pairs, each on one side of the packaging signal, and the use of cell lines that express the recombinase capable of recognizing the sequence pair, in order to excise the region localized between them and, thereby, the packaging signal. The use of helper adenoviruses to allow the multiplication of a gutless adenovirus in production methods for the latter which are based on a delay of the packaging moment of the helper adenovirus over that of the gutless adenovirus is new in the technique, as is the use of the presence of the attB sequence corresponding to ϕC31 bacteriophage to influence the packaging process of the adenovirus in whose genome the attB sequence can be found, without the need for that genome to also contain an attP sequence which also corresponds to ϕC31 bacteriophage. Therefore one of the aspects of the invention is the use of polynucleotide sequences that respond to the structure of an adenovirus genome, as reflected in the upper part of Fig. 1, and which in additionally comprise an attB sequence of ϕC31 bacteriophage located between the Ψ sequence and the end of the adenovirus genome closest to said Ψ sequence, to multiply gutless adenovirus, both if the polynucleotide sequence is placed in contact with the cell in which the packaged multiplication will occur in the form of a complete particle of adenovirus, as well as if the sequence constituting the genome does not form part of a complete particle of adenovirus and requires another mechanism different from the receptor-mediated endocytosis of the adenovirus in order to enter the cell, such as for example those mediated by agents with positive charge that facilitate entry of DNA molecules into the cell associated with them.

Initially, and by comparison with the strategies known until now, the authors of the invention considered necessary the presence of both an attB sequence and an attP sequence in the helper adenovirus genome, both flanking the packaging signal, in order to allow their excision. The exact localization in each of the two regions close to the packaging signal does not appear to be critical, and it seems possible that the attB sequence is located both 5' as well as 3' to packaging signal Ψ. However it has now been discovered that placing the attB sequence in 5' with respect to the packaging signal (that is, between the packaging signal and the ITR closest to it) has an unexpected effect, that of delaying the packaging moment of the genome containing the sequence. This effect does not require the presence of an attP sequence close to the other end of packaging signal Ψ, and allows the design of the method of the invention, based on the delay of the packaging moment of a helper adenovirus that contains an attB sequence of ϕC31 between the packaging signal and the ITR closest to it with respect to the moment of a gutless adenovirus whose genome is in the same cell. Therefore another aspect of the invention are the polynucleotide sequences that can be considered helper adenovirus genomes (that is, that comply with the conditions expressed above for a sequence to be considered an adenovirus genome and which comprise early and late protein coding sequences of the adenovirus under the control of promoters that allow their expression in eukaryotic cells), and which also comprise an ϕC31 attB sequence located 5' to the packaging signal. The complete viral particles in which these polynucleotide sequences are packaged are also comprised within the scope of the invention.

The invention will be now described in more detail by the following Figures and Examples.

## BRIEF DESCRIPTION OF THE FIGURES

Fig. 1 shows a drawing of the typical structure of an adenovirus particle. The genome is represented by a folded double band marked as "DNA GENOM." The proteins associated with each of the ends of same are marked as "Core TP" , while proteins marked as "Core VII" are other DNA-associated proteins that facilitate their packaging in the capsid. Also indicated are the capsid components: the pentameric structures located at the vertices of the icosahedron forming the capsid (labeled as "penton"), the fibres having their origin in said pentameric structures ("Fibre"), and the hexameric structures that form the rest of the capsid ("Hexon").

Fig. 2 shows the map of the serotype 5 adenovirus genome (upper part of the drawing, immediately below the scale indicating the number of base pairs, labeled as "Ad5"), as well as a map of the genomes of first generation (labeled as "1$^{st}$ Gn."), the second generation (labeled as "2$^{nd}$ Gn"), and gutless adenovirus (lower part of the drawing) vectors. The abbreviations "E1", "E2A", "E2B", "E3" and "E4" indicate the localization of the regions wherefrom the early adenoviral transcripts receiving the same denominations are transcribed; the presence of the "Δ" symbol in front of any of these abbreviations indicates that that region has been deleted in the genome over whose map the abbreviation is found. The abbreviations "L1", "L2", "L3", "L4", and "L5" indicate the localization of the regions wherefrom the late adenoviral transcripts receiving the same denominations were generated. MLP: Major late promoter. Ψ: Packaging signal.

Fig. 3 shows a drawing of the process through which the adenovirus particles penetrate the cell ("Cel.") via receptor mediated-endocytosis, favoured by the integrins that interact with the penton proteins after having produced the protein interaction of the fibre with an adequate receptor in the cell membrane. Once internalized in an endocytic particle, the viral particles are released in the cytoplasm, giving rise to a spheric particle that lacks pentons, which endocytic particle migrates towards the nucleus ("Nucl."), where the genomic DNA is released.

Fig. 4 shows the process to obtain gutless adenovirus particles using a helper adenovirus whose packaging signal Ψ is flanked by IoxP sequences, by producing the cotransduction and multiplication of adenovirus genomes in cells expressing the Cre recombinase, 293/Cre.

Fig. 5 shows a drawing of each of the adenoviral genomes used in the examples of the present application.

Fig. 6 shows the band pattern obtained upon digesting the plasmic PKP1.4ΔCMVΔMCSattBloxPΨGFPattP with the restriction enzymes indicated over each of the lanes. The central lane, marked as "1 kb Marker", corresponds to the lane in which a size marker was run which consists of a ladder of bands, with each one 1 kb larger than the previous one.

Fig. 7 shows the comet effect observed after 96 hours post-transduction of the nucleotide sequence corresponding to Ad5/FC31 by dispersion of the viral load upon lysis of the adenoviruses produced by the cell and giving rise to an intense point of cells, expressing protein GFP and a decreasing gradient of expression.

Fig. 8 shows the viral cycles of adenoviruses Ad5/attP (Fig. 8a) and Ad5/IoxP.FC31 (Fig. 8b), as a percentage of the virus isolated after the number of hours post-transduction indicated in abscises, either from cells (PLL), or from culture supernatants (SN), calculated in all cases with respect to the maximum total finally obtained of that adenovirus.

Fig. 9 shows comparative graphs of the cell cycles of the different helper adenoviruses assayed that are indicated on the right to the graph, as a percentage of the virus isolated after the number of hours post-transduction indicated in abscises.

Fig. 10 shows a blotting in the form of dots ("Dot-blot") of the DNA extracted from HEK293 cells infected with adenovirus Ad5attP (left part of the graph) or with adenovirus Ad5/FC31 (right part of the graph), after the number of hours post-transduction indicated at the top of each of the columns. The lower line shows a spot intensity pattern in the form of dots that correlate the intensities obtained with the amounts of DNA, in nanograms (ng) indicated immediately below.

Fig. 11 shows a bar chart representing the infective units detected per cell (IU/cel) in cells infected with Ad5/attP, Ad5/FC31 and Ad5/GFP after 36 hours (bars filled with vertical lines:||) or 56 hours (darkened bars: ) from entry of the viral genome into HEK293 cells. The arrows located at the right of the chart delimit the areas of viral amplification (upper arrow, the longest one) or non amplification (lower arrow, the shortest one).

Fig. 12 show the effect of the *in trans* supply of viral proteins with respect to the packaging capacity of adenovirus Ad5ϕC31.IoxP from the production of infectious units (IU) per cell of the different adenoviruses evaluated 36 hours after infection of HEK293 cells at a multiplicity of infection of 1. The three bars in the group on the left, marked as "indiv", show production of adenovirus Ad5attP (bars with vertical lines: ||), Ad5ϕC31.IoxP (bars with horizontal lines: =) or Ad5Bgal (dark coloured bars: ), infected individually. Bars in the intermediate group, marked as "IU-GFP+AD5Bgal"), show infectious units produced per cell expressing the protein GFP, when coinfected with Ad5attP+Ad5Bgal (bar filled with vertical white lines over dark background: ) or Ad5ϕC31.IoxP + Ad5Bgal (bar filled with horizontal white lines over dark background: ). Bars in the group on the left, marked as "IU-Bgal + Ad5Bgal" show infectious units produced per cell expressing β-galactosidase, when coinfected with Ad5attP+Ad5Bgal (bar filled with vertical black lines over dark background: ) or Ad5ϕC31.IoxP + Ad5Bgal (bar filled with horizontal black lines over dark background: ).

## EXAMPLES

Production of gutless Ad requires the generation of a helper Ad that can supply the viral proteins and in turn be eliminated during the production process. The following examples describe the procedures used to generate helper adenoviruses with different combinations of sequences recognized by specific recombinases and tests performed with them to verify their potential as helper.

Tests were carried out using the methodologies described below:

Amplification and purification of plasmid DNA.

Minipreparations of DNA were prepared by alkaline lysis from 3 mL of culture to obtain plasmid DNA in small amounts (5-15 μg). This method is based on lysis of bacteria from an alkaline buffer, following by a protein precipitation with potassium acetate and a later precipitation of the plasmid by isopropanol and a final wash with ethanol at 70%. RNA is eliminated through RNAse A.

- Resuspension solution: 50 mM Tris-Cl a pH 8.0; 10 mM EDTA; 100 μg/mL RNAseA.
- Lysis solution: 200 mM NaOH; 1% SDS (w/v).
- Protein precipitate solution: 3.0 M potassium acetate pH 5.5

Purification of DNA fragments

DNA is purified in agarose gels using the QBIOgene GENECLEAN Turbo Kit (Irvine, California, USA), following the manufacturer's protocol.

Restriction, binding and alkaline phosphatase enzyme

Enzymes used in this work were FERMENTAS and NEW ENGLAND Biolabs brand. 5U of digestion were used for 2 μg of plasmid DNA under temperature and solution conditions established by the manufacturer. Digestion time varies from 1-18 hours based on the administered dose. Alkaline phosphatase and ligase were used in the doses and times established by the manufacturer.

attP region sequencing

All sequences analyzed were sequenced in the Sequencing Service of the Universidad Autonoma de Barcelona School of Veterinary Medicine. The attP region was sequenced with the following oligonucleotide from the pBCPB+ plasmid:
6600R2440: 5' CACCTCCCAGATCCTTATCGA 3' (SEQ ID NO:5).

Region attBloxP Proof

Region attBloxP was tested through sequencing with the following oligonucleotides:

In the cloning plasmid:

SP6: 5' ATTTAGGTGACACTATAGAA 3' (SEQ ID NO:6)
T7: 5' TAATACGACTCACTATAGGG 3' (SEQ ID NO:7)

In the final plasmid:

Ad5140-160: 5' CGGAACACATGTAAGCGACGG 3' (SEQ ID NO:8)

Mutagenesis addressed against the target SgrAI

The oligonucleotides used were as follows:

MutDIRAgel: 5' C**ACCGGT**GTACACAGGAAGTGACAA 3' (SEQ ID NO:9)
MutREVAgel: 5' C**ACCGGT**GTACACACCAAAAACGTC 3' (SEQ ID NO:10)

The following PCR (polimerase chain reaction) conditions were used:

45 µl de MIX high fidelity (INVITROGEN Platinum PCR Supermix high fidelity).
10 pmol of each primer.
10 µg of plasmid DNA
To 50 µl of milliQ water.

Reaction cycles: 94°C 1 min, (95°C 30 sec, 51°C 30 sec, 68°C 7 min) 15 cycles, 68°C 7 min and 25°C 5 min.

After the PCR reaction 10 U of Dpn I enzyme was added, which degrades bacterial DNA, for 1 hour at 37°C + 5,6 µl of Dpn I buffer.

Transformed into competent cells and colonies were grown for later proof by digestion pattern.

The region of interest was sequenced using the following oligonucleotide to proof the introduction of the target Age I:

Ad5140-160: 5' CGGAACACATGTAAGCGACGG 3' (SEQ ID NO:8)

Amplification of attB and loxP sequences via PCR

Oligonucleotide attB_dir:

5'TTATAAAGGTACCCACCGGTCCGCGGTGCGGGTGCCAGGGCGTGCCC
TTGGGCTCCCCGGGCGCGTACTCCAC**GCGGCCGC**AT3' (SEQ ID NO:11)

Oligonucleotide attBloxP_rev:

5'TTATAAACACCGGTC**GCGGCCGC**ATAACTTCGTATAATGTATGCTATACG AAGTTAT**GCGGCCGC**GTGGAGTACGCGC3' (SEQ ID NO:12)

(Underlined and bold sequences of both nucleotides corresponding to Nott sequences; underlined sequences not in bold corresponding to AgeI sequences)

A band of 140 bp was amplified with these oligos through 30 PCR cycles.

0.5 μl oligonucleotide attB_dir with target AgeI and Not I attB
0.55 μl oligonucleotide attBloxP_rev:
5 μl buffer Taq
4 μl $MgCl_2$ (25 mM)
0.4 μl dNTPs (25 mM)
0.3 μl Taq Pol (Promega) 5U/μl
39.85 μl milliQ water

Conditions: 94°C, 30 sec, (94°C 30 sec, 54°C 30 min, 74°C 30 sec) 30 cycles, 74°C, 2 min y 25°C 2 min.

Introduction of loxP sequence flanking the Ψ signal at 5'.

The following oligonucleotides were used to introduce the loxP sequence :

loxP NotI/AvrII DIR(peGFP-C1):

5' TAGCGAAT**GCGGCCGC**T**ATAACTTCGTATAGCATACATTATACGAAGTTAT** CCTAGGTGACTGGGCACAACAGACAAT 3' (SEQ ID NO:13)

NotI/AvrII REV(peGFP-C1):
5'GCATATCA**GCGGCCGC**TCCTAGGGTATCGACAGAGTGCCAGC 3'(SEQ ID NO:14)

(Underlined and bold sequences of both oligonucleotides with 8 nucleotides correspond to NotI sequences; underlined and bold sequences of 6 nucleotides correspond to AvrII sequences. The intermediate sequence appearing in bold but not underlined in oligonucleotide loxP NotI/AvrII DIR(peGFP-C1) correspond to the loxP[33] sequence).

PCR conditions to amplify the 1 Kb band with loxP sequence were:

2 μl (5 ng) of plasmid peGFP-C1
0,4 μl dNTPs (25 mM)
0.3 μl Taq Pol (Promega) 5U/μl
4 μl $MgCl_2$ (25 mM)
5 μl buffer Taq Pol 10X.
1 μl (10 pmol) oligonucleotide loxP NotI/AvrII DIR(peGFP-C1)
1 μl (10 pmol) oligonucleotide NotI/AvrII REV(peGFP-C1)
37.3 μl milliQ water

PCR Conditions: 95°C 1 min, (95°C 30 sec, 95°C 30 sec, 74°C 1 min 15 sec) 10 cycles, (95°C 30 sec, 60°C 30 sec, 74°C 1 min 15 sec) 25 cycles, 74°C 2 min and 25°C 2 min.

TA cloning

During the PCR reaction cycles, the Taq polymerase normally introduces an A in the final region of the amplified fragment. This process normally occurs in the last amplification cycles. For this reason a digested plasmid containing a T in the ends is used to clone fragments amplified by Taq Pol. In this way the amplified fragment can be bound with this plasmid. PROMEGA pGEMT-easy vector plasmid is used, allowing a selection by colour of positive colony, which will appear white as opposed to the negative colonites, which appear blue, thus assuring the selection of the colonies to be amplified.

Homologous recombination.

Plasmid pKP1.4 is linearized with SwaI to prepare the homologous recombination, and the adenoviral launch plasmid used to recombine with 1 or 2 enzymes outside of the recombination zone of interest. 50 ng of pKP1.4-SwaI are mixed with 50, 100 and 150 ng of linearized launch plasmid and transformed into Rec+ (BJ5183) cells. Digestion of the amplified colonies is tested. Clones with a high molecular eight are transformed into strain TOP10 of *Escherichia coli*, allowing greater yield. The digestion pattern is tested with different enzymes until a positive clone is selected. Maintain DNA at -20°C or -80°C inasmuch as the plasmid easily degrades at room temperature.

Preparation of the adenoviral genome

In order to replicate the adenoviral genome in a cell, it is essential that it be in linear form and with ITR sequences immediately before the ends; as such the plasmids comprising the adenovirus genomes used in the examples must be linearized prior to transducing the DNA sequences corresponding to the genomes, eliminating all additional bacterial sequences and leaving the ITR sequences precisely at the ends of the lineal fragment generated. The following examples describe the linearization of the adenoviral plasmid in the target Pac I to eliminate the replication origin of the plasmic and ampicillin-resistant gene. This was done by digesting 100 μg of DNA with 50 U of Pac I in a final volume of 200 μl during 14-16 hours. Precipitate through 20 μl of potassium acetate 3M pH 5.2, 550 μl of cold absolute ethanol and incubate 30' at - 80°C. Centrifuge at 14000 rpm and 4°C discarding the supernatant. Wash DNA with ethanol at 70% and let dry for 15'. After this the DNA was resuspended in milliQ water or TE (10 mM/a mM) pH=8, incubating it for 1 hour at 37°C.

Purification of viral vectors

Once the cells are re-suspended in the remaining supernatant medium (40-45 mL), two serial centrifugations were performed in a CsCl (Cesium Chloride) gradient. The first is with a CsCl gradient with 2.5 mL of 1.4 g/mL density stock, and the others with 2.5 mL of 1.25 g/mL stock. The virus fraction is then gently added to the gradient. It is centrifuged in a SW40 rotor for 1.5 hours at 35,000 rpm and 18°C. This produces a relatively broad band containing the formed capsids, the empty capsids, and the remnants of viral proteins. In the second centrifugation, a 1.34 gr/mL gradient is used, which is approximately the same density as the formed virion. Using this technique, 3 bands are normally observed. One of higher density with the capsids assembled with DNA and two lower-density bands corresponding to the empty capsids and viral proteins. Once the virus is centrifuged, the band with the DNA-bearing capsids is extracted with a hypodermic needle, and a Sephadex column is used to eliminate the remaining CsCl (which is toxic to the cells).

Determination of the cell cycle

The cell cycle for the various Ads is carried out on a 24-well plate with a MOI=5, using the adenovirus Ad5ϕC31.loxP.1, Ad5AttP.1, and Ad5GFP, repeating the experiment 3 times (n=3) for each of the adenoviruses. Samples are collected between 24-40 hours and between 48-64 hours. There is little or no production of the vectors prior to 24 hours. After this, the supernatant is collected separately, and 500 to 1000 μl is added to collect the cells.

Titration of the adenoviral vectors

The titration of the adenoviral vectors was accomplished by limiting dilution on a 96-well plate with HEK293 cells when they reach 80% confluence. This is usually performed in triplicate to minimize errors.

It is performed with dilutions on 24- and 96-well plates, sequenced from $10^{-3}$ to $10^{-10}$. 100 μl of the dilutions are deposited on previously aspirated 96-well. Because all the Ads used express the GFP gene, their titration was performed using fluorescence microscopy.

Titration of the cell cycle

Samples of the cellular well on the 24-well plate were separated into supernatant and cells. The supernatant was collected directly from the well, and between 500 and 1000 μl of fresh medium was added to collect the cells. This fraction was subjected to 3 freeze/thaw cycles to release the intracellular virus, and these were subsequently titrated separately. Because the number of samples was very high (>500), 0.5 μl of the sample was used directly on the 96-well. Normally, with this many samples one can see from 0.5% up to 100% of cells infected. This allows the sigmoid growth curve displayed by these vectors to be seen easily.

Titration of productions at 36 and 56 hours.

The titration of the production experiments was performed by limiting dilution. 15 μl from each sample was diluted in 135 μl of medium, and from there 35 μl in 115 μl, and so on. 100 μl from each dilution was deposited on a 96-well plate with HEK293 cells at 80% confluence. The number of green cells seen via fluorescent microscope under UV light was counted. Using this method, the following formula was derived, with can be used to determine the number of infectious units/cell.

$$\text{No. IU/cell} = \frac{\text{No. of green cells (GFP+)} \times 4^{(n-1)} \times \text{Volume of sample collected } (\mu l)}{100{,}000 \text{ cells in a 24-well plate}}$$

FACS analysis

The FACS analysis of the various Ads was conducted in 24-well plates using the Ad5/loxP.FC31, Ad5/attP, and Ad5/GFP adenoviruses, with n=2 and 30% infection. Samples were collected at 24 and 36 hours. Then, the cells were washed with PBS 1 % and were fixed in 2% paraformaldehyde. Once the cells were fixed, they were analyzed and quantified in FACS service of the IBB-UAB institute.

**Example 1.- Production of *helper* adenovirus (Ad *helper*)**

The strategy followed to generate *helper* Ad was the introduction of the attB/attP sequences flanking its packaging signal and a GFP (*green fluorescent protein,* a protein which produces a characteristic green glow when observed under ultraviolet light) marker gene on this signal's 3' end for easy analysis of the potential of these vectors.

The production of *helper* Ad was based on the human serotype 5 (Ad5) adenoviral genome, which can be accessed in the GenBank database under code AC_000008. The Ad5 adenoviral genome is inserted in the pKP1.4 plasmid, which contains the entire viral coding region, except for part of the E1 region and part of the E3 region (the 28250 to 30757 nucleotides for the Ad5 genome sequence shown in GenBank). It also contains the CMV promoter, a multiple cloning site (MCS) that allows for the incorporation of genes, and the polyA for SV40. To clone the signal or therapeutic genes, a shuttle plasmid called pTG6600, which is 6.2 Kb in size, was used to start with, instead of using the entire pKP1.4 plasmid, which is 34 Kb. The pTG6600 plasmid contains the initial 4262 nucleotides and the region of ampicillin resistance, and the origin of replication for the pKP1.4 plasmid.

The plasmids pTG6600 (obtained from the Genethon-III Gene Therapy laboratory in France), peGFP-C1 (Clontech), pBCPB+ (MTA: Material Transfer Agreement from Michele P. Calos), pKS-RSV/GFP (sent by Eric Kremer, University of Montpellier), pGEMT-easy (Promega), pKP1.4 (MTA Eric Kremer) and pKP1.4ΔCMV (MTA from Eric Kremer) were used in this task.

The *helper* adenovirus was produced using the techniques listed at the beginning of the "Examples" section, following these steps:

**1.1 Preparation of the shuttle plasmid pTG6600**

The first modification step for the pTG6600 plasmid was the specific deletion of MCS and polyA from SV40, as this area was not needed for cloning. To achieve this, the pTG6600 plasmid was digested through the Mfe I and EcoR I targets to eliminate the 183 bp band from the MCS, producing 3 fragments of 5310, 616, and 183 bp. The 5310 and 616 bands were reattached to obtain the pTG6600ΔMCS plasmid.

**1.2 Introduction of the attP sequence flanking the Ψ signal at 3' .**

Next we used the plasmid pBCPB+, which contains ϕC31 recombinase attP sequence (SEQ ID NO:3) as the attP region, flanked by sequences that are contiguous with it in the ϕC31 bacteriophage genome, consisting of the entire attP sequence of 218 nucleotides (SEQ ID NO:4) introduced and flanked by Spe I targets to introduce it into the pTG6600ΔMCS plasmid. The pTG6600ΔMCS plasmid was digested by the Spe I and Nhe I targets, thus eliminating the CMV promoter, and the 221 bp band from pBCPB+ digested with Spe I was introduced to generate the pTG6600ΔMCSΨattP plasmid. Once the attP sequence was introduced, the region was sequenced to prove that the attP nucleus was correct. It was then noted that the sequence introduced had undergone a mutation of 1 nucleotide

outside the ϕC31 recombinase activity region, which was not mutated, and as a result we continued to follow the established strategy.

### 1.3 Introduction of a GFP expression cassette to facilitate the analysis of virus production.

The reasoned strategy for observing these vector's jump of the packaging signal during production was the introduction of a GFP (green fluorescent protein) cassette, which would be flanked by the attB/attP signals. It was therefore decided to introduce this cassette in the 3' region of the packaging signal. To accomplish this, pKSRSVGFP plasmid, which contains the GFP gene controlled by the RSV promoter and a polyA of SV40, was used. The pKSRSVGFP and pTG6600ΔMCSattP plasmids were digested by the Sal I and Spe I targets, and the 1597 bp GFP cassette was extracted and introduced specifically immediately after the packaging signal, namely at 54 nucleotides from the end, on nucleotide 454 of the Ad5 sequence, thus producing the pTG6600ΔMCSΨGFPattP plasmid.

### 1.4. Introduction of the attB sequence flanking the Ψ signal at 5'

Once the abovementioned sequences were introduced, it was decided to introduce the attB sequence between the Ad 5' ITR and the packaging signal. As has been noted above, the attB sequence of the ϕC31 bacteriophage has a length of approximately 500 bp, even though recent studies have shown that it has a minimum region of 34 bp[21] comprised of a nucleus at the TTG nucleotides and a region rich in cytosines (C) and guanines (G) flanking the nucleus. Because the introduction of sequences between the ITR and the packaging signal reduces the packaging capability of the adenoviral genome[35], it was decided to use a minimal attB region of 53 bp instead of the entire attB sequence of approximately 500 bp.

The first strategy designed was the direct cloning of the attB sequence in the 189 nucleotide of the 5' end of the Ad, right on the SgrA I target. Having observed the *star* activity of for this enzyme (which, under certain reaction conditions is able to recognize as targets to cut sequences that do not match exactly its most common recognition sequence), it was decided to make a mutagenesis directed against the SgrA I target (5'CPuCCGGPyG3') and reconvert it into the Age I target (5'ACCGGT3'). To accomplish this, two oligonucleotides were designed in order to introduce the new target. Then, to confirm the process of directed mutagenesis, a variety of verification digestions were performed, the region was sequenced with the Ad5140-160 oligonucleotide for greater assurance, and the insertion of the Age I target was confirmed. The pTG6600(**AgeI**)ΨGFPattP plasmid was produced using this method.

Once the 3' region of the packaging signal was modified (introduction of the GFP cassette and, if necessary, the attP sequence, in the plasmid vectors), the attB was introduced using PCR. To accomplish this, two oligonucleotides were designed that carried the attB sequence in the forward oligonucleotide (attB_dir, SEQ ID NO:11) and the loxP sequence in the reverse oligonucleotide (attBloxP_rev, SEQ ID NO:12). Using this technique, the attB sequence remained inserted together with nucleotide 193 from the Ad5 sequence. In turn, the loxP sequence of the Cre recombinase was introduced with the intention of creating new *helper* Ads that could be used in Ad-producing cell lines that would express the Cre recombinase. This loxP sequence was introduced flanked by the Not I sequences for future excision.

Once the attBloxP band was amplified, the 140 bp sequence in Promega's pGEMT-easy plasmid was cloned. The oligonucleotides SP6 and T7 were sequenced, and it was noticed that the introduced targets and the attB sequence did not undergo changes, but the loxP sequence had undergone mutations, for which reason it was decided to clone the enter fragment in the pTG6600ΔMCS(AgeI)ΨGFPattP plasmid and excise the loxP sequence, digesting the construct with the Not I target. This technique was used to obtain the pTG6600ΔMCSattBΨGFPattP construct.

### 1.5 Introduction of the loxP sequnce flanking the Ψ signal at 5'.

Because the loxP sequence had undergone mutations, it was decided to re-introduce the loxP sequence with the loxP NotI/AvrII_DIR(peGFP-C1) and NotI/AvrII REV(peGFP-C1 oligonucleotides using a different cloning strategy. It was decided to amplify one kilobase of an irrelevant plasmid flanked by the targets Avr II, which is not found in any of the constructs described so far. The loxP target and the kilobase were flanked by Not I targets, which was used for cloning the region of interest. To accomplish this, the abovementioned oligonucleotides were designed.

The irrelevant kilobase came from the peGFP-C1 plasmid, together with the loxP and the introduced targets. The amplified band was cloned using the *TA cloning* method on PROMEGA's pGEMT-easy plasmid, and was sequenced to see if any mutations had occurred. Once it was shown that the loxP sequence had not undergone any mutations, the pGEMT-loxP(NotI/AvrII) plasmid was digested with the Not I enzyme, extracting the amplified kilobase. The pTG6600ΔMCSattBΨGFPattP plasmid was opened using the Not I target, and the kilobase was cloned to create the pTG6600ΔMCSattB**loxP(1Kb)**ΨGFPattP plasmid. Once the irrelevant kilobase was cloned, it was deleted by digestion with Avr II and later reattached to finally create the pTG6600ΔMCSattB**loxP**ΨGFPattP plasmid .

**1.6 Generation of the genomes for *helper* Ad by homologous recombination.**

Once these constructs were created, a homologous recombination process was conducted on the bacteria by incorporating the modified regions in the pKP1.4 plasmid, which contains the entire viral region of the serotype 5 Ad genome. To accomplish this, the various constructs (pTG6600ΔMCSattBIoxPΨGFPattP, pTG6600ΔMCSΨGFPattP, pTG6600ΔCMVΔMCSattBΨGFPattP, pTG6600ΔCMVΔMCSattBIoxPΨGFPIoxP, pTG6600ΔCMVΔMCSattBIoxPΨGFPIoxPattP) were digested using the Fsp I enzyme, which is found outside the region of interest. Also, the pKP1.4ΔCMV plasmid was digested with Swa I. Both digestions were transformed in the bacterial strain BJ5183, which allows the homologous recombination for the creation of the modified adenoviral genome. After the transformation, colonies were grown and the digestion pattern of the plasmids was verified using various restriction targets. Due to the low performance of the plasmid DNA minipreparation with this strain, the positive clones were then transformed in the TOP1 strain of *Escherichia coli*, which produces higher yields. Once the positive clones were obtained, they were amplified into plasmid DNA maxipreparations, which allowed a sufficient quantity of plasmid to be obtained in order to transduce cells and generate the Ad.

Using this technique, the following plasmids were created, which comprise the sequences that correspond to the adenoviral genomes that contain distinct sequences that are recognized by recombinases in a variety of locations, as is shown in Fig. 5:

PkP1.4ΔCMVΔMCSΨGFPattP → **Ad5/attP** (Ad *helper* control)

PkP1.4ΔCMVΔMCSattBΨGFPattP→**Ad5/FC31**

PkP1.4ΔCMVΔMCSattBIoxPΨGFPattP→**Ad5/loxP.FC31**

PkP1.4ΔCMVΔMCSattBloxPΨGFPloxP → **Ad5/attB.Cre**

PkP1.4ΔCMVΔMCSattBIoxPΨGFPIoxPattP →**Ad5/FC31.Cre**

Each of these genome sequences, shown in Fig. 5, contains the human serotype 5 ITR at its ends, as well as the packaging signal (Ψ) and the entire coding region of the aforementioned adenovirus genome, with the exception of the E1 region and parts of the E3 region (the part that up to 28250 to 30757 nucleotides in the sequence corresponding to the genome of that serotype present in GenBank). The rest of the elements in this figure that are present in the genomes shown are as follows:

- attB: sequence represented by SEQ ID NO:2, which corresponds to a fragment of the complete attB sequence recognized by the ϕC31 recombinase in the bacteria's genome, which includes the minimal functional sequence of 34 bp recognized by this recombinase;
- attP: sequence represented by SEQ ID NO:4, which contains the sequence that recognizes the ϕC31 recombinase in the ϕC31 bacteriophage genome (nucleotides 72 to 155 of SEQ ID NO:4), and the flanking sequences of the same bacteriophage genome;
- IoxP: sequence represented by SEQ ID NO:12, which contains the sequence recognized by the Cre recombinase (nucleotides 24 through 57 of SEQ ID NO:12);
- GFP: expression cassette for the green fluorescent protein, with the RSV promoter and polyadenylation signal for the SV40 virus.

In addition to the aforementioned adenovirus, it was decided to use a control Ad with a GFP expression cassette controlled by the CMV promoter, the Ad5/GFP adenovirus, which does not carry any sequence recognized by recombines.

**1.7 Analysis of the stability of the PKP1.4ΔCMVΔMCSattBloxPΨGFPattP plasmid**

Once the plasmid is generated, the band patterns that these vectors exhibited was analyzed by different restriction targets to control for reorganizations and to ensure the stability of the adenoviral genome. Fig. 6 shows the band pattern corresponding to the plasmid indicated and shows that the genome is stable and has not suffered internal recombination, because the observed patterns matches those expected. Once the sequences were introduced in the final vector, the 5' region of the Ad was sequenced between nucleotide 180 and nucleotide 600 for greater assurance. The same analysis was repeated for the various constructs.

The sequencing of the region that includes the attB sequence led to the sequence represented by SEQ ID NO: 16, in which the part corresponding to the adenovirus genome is represented by SEQ ID NO:15.

## Example 2. Amplification of adenovirus Ad5/attP.-

### 2.1 Production of Ad5/attP.

Prior to the production of the vector, the viral genome to be transduced needs to be prepared. The pKP1.4ΔCMVΔMCSattBloxPΨGFPattP plasmid contains the gene for ampicillin resistance and its origin of replication, which must be eliminated. This region is flanked by 2 Pac I targets. To accomplish this, 100 μg of plasmid was digested with the Pac I enzyme, and it was purified for subsequent transduction in HEK293 cells. In this way, the plasmid's Amp+ori region was eliminated and the viral genome was opened, producing a linear molecule, which is the form that the Ad genome takes when it enters the cell nucleus.

To amplify the adenoviral genomes, a transduction was performed with PEI (polyethylenimine of 25000 Da average molecular weight, supplied by Aldrich, ref.= 40,872-7) in a 6-well plate. Once the pTG6600ΔMCSattBloxPΨGFPattP and pTG6600ΔMCSΨGFPattP plasmids were transduced, at 24 hours the expression of the GFP gene could be observed under UV light, and the percentage of transduced cells could be counted. The transduction is considered good when at least 30% of the cells are transduced.

### 2.2 Purification and titration of Ad5/attP

After 72 hours, once the Ad has gone through 2 36-hour viral cycles, the cells and supernatant were collected together, and 3 freeze/thaw cycles were conducted to release the virus contained in the cell nucleus. All content was centrifuged to remove the cells, and once the supernatant was collected, it was used to amplify the virus in successive steps until 20 15 cm-plates were produced in 36 hours. Once the cells were re-suspended in the supernatant, 2 serial centrifugations were conducted using CsCl gradient. When using this technique, 3 bands are normally observed: one band of higher density, where the capsids assembled with DNA are found, and two lower-density bands corresponding to the empty capsids and viral proteins. The higher density band was extracted, and remaining CsCl was eliminated from the sample using a Sephadex column.

The Ad titration was performed using a limiting dilution which enabled a final dilution containing only a single virus to be obtained, which permitted the number of infectious units in the production to be counted. Physical particles is a value that tells us the total number of viral capsids with the viral genome that we have.

## Example 3. Amplification of different constructs of *helper* Adenovirus

The adenoviral vectors derived from Ad5 usually have a 36-hour cycle. To achieve this, in the final production, the cells are collected at 36 hours, so that most of the mature virion produced is found in the cell nucleus, just before cell lysis. This will concentrate the cells for further purification.

When the various plasmids are transduced and the producing cells are collected, it was noted that the Ad was not amplified in successive steps. After repeating this process up to 4 times, it was thought that the Ad was not functional, even though it expressed the GFP protein in the cells. Even though a correct digestion band was observed, it was thought that some essential gene of the Ad cycle had mutated. However, another option was that the percentage of the initial transduction was low and that this would lead to a slower viral cycle. To determine if this was the cause, the number of process hours was increased to 96. Surprisingly, under UV light it could be seen that the Ad created was functional, because the comet effect was observed (see Fig. 7). The comet effect is observed when the Ad, after cell lysis, scatters its viral load in the direction of the plate, so that there is an intense point of cells expressing GFP protein and a gradient of decreasing expression.

After various transduction tests, it was noted that the Ad Ad5/FC31 had a viral cycle of between 48 and 60 hours. Once it had been repeatedly observed that the Ad generated had a slower viral cycle, it was decided to create this vector, allowing it 2 amplification cycles (5 days). Using this technique, it was possible to obtain an Ad production that allowed various tests to be conducted and to amplify it until a viral preparation was obtained with elevated vector titers.

## Example 4. Determination of the viral cycle of the control Ad Ad5/attP and Ad5/GFP and the various *helper* Adenoviruses

To produce the various *helper* adenoviruses with high titers, the exact hour when the adenovirus cell lysis occurred needed to be known. To accomplish this, an experiment was designed to discover the Ad production in supernatant and cells at different times. Samples were collected every 4 hours to observe the production curve of Ad in 293 cells.

To conduct the Ad viral cycle experiment, HEK293 cells on 24-well plates were infected at an MOI=5. MOI=5 (i.e., multiplicity of infection, which is an indication of the number of infectious viral units per cell) was chosen due to the fact that this MOI can infect 70-80% of the cells. In a first experiment, samples were collected from 4 to 72 hours with a 4-hour time difference and n=3. To avoid constant 24-hour monitoring, it was decided to use 2 timelines. The first timeline started at time 0 and the second timeline at +12 hours. To prevent the timeline from producing a larger or smaller amount of virus due to cell confluence, the intermediate points of 24, 36, 48 and 60 were duplicated on the two timelines to validate the experiment. The cells and supernatants were analyzed on a 96-well plate, and using this, the first approximation of the cell cycle behaviour of these Ad were obtained. In the first experiment, neither the Ad5/loxP.FC31 nor the Ad5/attP produced viral particles after 16 hours, which corresponds in both cases to a normal kinetics, since it is difficult to detect viral particle production before 16 hours, and they completed their viral cycle completed before 64 hours. In terms of the moment when the viral cycle was completed, the Ad5/attP and Ad5/GFP seemed to follow a normal 36-hour cycle. The experiment was repeated on 4 different days, obtaining similar graphs in each case. As is seen in both graphs, the Ad levels in the supernatant (S) follow the cell levels (PLL) in terms of production levels, with a time difference of 3 to 6 hours (Fig. 8a and 8b).

The virus remained retained in the nucleus until the last moment of the cycle, which is when the cells underwent lysis and released the new virions into the medium. For this reason, viral production stage in the cell phase is the most meaningful date to compare the kinetics of the various viruses. Upon analyzing the *helper* Ad and control cells at different times, it was noted that, at an MOI of 5, the Ad5/attP and Ad5/GFP had similar kinetics, with a maximum at 36 hours, while the rest of the *helper* Ad with ϕC31 attB/attP sequences had slower growth kinetics, and their viral cycle increased up to 50-56 hours (Fig. 9).

**Example 5. Protein production of Ad5/loxP.FC31, Ad5/attP, and Ad5/GFP.**

At that time, due to the reproducibility of results and the high number of verifications, it was decided to continue the experiments with only one *helper* Ad that was representative of all of the of *helper* adenoviruses: Ad5/loxP.FC31. One of the primary functions of *helper* Ad is to support the viral proteins for the packaging of the *gutless* Ad. After noting that the viral cycle of Ad5/loxP.FC31 was delayed 14-20 hours in comparison with a control Ad (Ad5/attP and Ad5/GFP), it is relevant to know if the *helper* Ad could support the viral proteins during the entire cell cycle. To find out if it the Ad5/loxP.FC31 *helper* Ad protein synthesis kinetics was affected in its viral cycle or whether it was capable of producing the same amount of protein as a control Ad, an analysis of GFP protein synthesis was conducted at 24 and 36 hours by FACS (Fluorescence Activated Cell-Sorter). HEK293 cells were infected with the *helper* Ad and controls at 80% confluence on 24-well plates, at MOI=5. A condition of 30% infection was used. Samples were collected at 24 and 36 hours, and the results obtained are shown below in Table 1:

**Table 1.- Fold change of Increase in GFP protein synthesis between 24 and 36 hours.**

| VIRUS | 24-36 hours (relative units) |
|---|---|
| Ad5/attP 30% | 3.07 |
| Ad5/loxP. FC31 30% | 3.78 |
| Ad5/GFP 30% | 2.59 |

As noted in Table 1, *helper* Ads' expression of the GFP protein has a kinetics similar to the control Ads. The data indicates that the *helper* adenovirus genome of the invention is not retained in a nuclear region with low or no protein expression.

**Example 6. Kinetics of viral genome replication**

One of the most relevant aspects in determining possible causes for the slow-down of the viral cycle is determining if the replication process of the viral genomes is affected. To rule out this possibility, it was decided to study and compare the replication of the Ad5/attP and Ad5/loxP.FC31 adenovirus genomes. To accomplish this, HEK293 cells were infected at an MOI=5 and samples were collected at 24, 28, 32 and 36 hours. Viral DNA was extracted and counted by the Dot-Blot method (transfer of dot-shaped molecules).

As the Dot-Blot test demonstrates, and as is shown in Fig. 10, the replication kinetics for the Ad5/loxP/FC31 genome is not affected and is similar to that of the control, Ad5/attP.

We also analyzed the infectivity of the adenovirus for each of the times studied in the Dot-Blot and, as expected, a significant reduction (99%) in the number of infectious particles of Ad5/loxP.FC31 was noted with respect to control, Ad5/attP. The specific data obtained for each time are those shown below in Table 2:

**Table 2.- Infected units per cell and quantity of DNA extracted at different infection times for Ad5/attP and Ad5/loxP.FC31**

| | Time (hours) | | | |
|---|---|---|---|---|
| | 24 | 28 | 32 | 36 |
| Infective units per cell (IU/cel) | | | | |
| Ad5/attP | 554.20 | 453.37 | 7797.13 | 4839.59 |
| Ad5/loxP.FC31 | 0.46 | 4.20 | 14.70 | 42.07 |
| Number of viral DNA (ng) | | | | |
| Ad5/attP | 3.84 | 5.15 | 7.98 | 14.38 |
| Ad5/loxP.FC31 | 4.32 | 8.67 | 10.11 | 14.09 |

Looking at the results indicates that although there are approximately the same number of genome copies in the cell, only 1% of the *helper* adenovirus Ad5/loxP.FC31 is packaged.

**Example 7.- Production of Ad5/loxP.FC31, Ad5/attP.and Ad5/GFP at 36 and 56 hours**

To understand Ad5/loxP.FC31's potential as a *helper* Ad, its production level in producer cell lines must be known. The *gutless* Ad described up to this point, because they are serotype 5, have a viral cycle of 36 hours in the HEK293 cell line. For this reason, it can be supposed that if the viral proteins are supplied, the virions formed will not have any problem with packaging and will be able to follow a normal cycle. Because the invention's *helper* Ad has a viral cycle that is very different in terms of production time with respect to the control virus, it was decided to look at the production of different vectors at 36 and 56 hours to see the differences in their production in Ad-producing lines (HEK293).

To accomplish this, the HEK293 cells grown to 75% confluence were transduced with the Ad Ad5/loxP.FC31, Ad5/attP, and Ad5/GFP at an MOI=5 on 24-well plates. With this infection level, the Ads give a transduction efficiency of 70-80% (observation under fluorescent microscope). The experiment was conducted with an n=4, and the Ad were incubated for 36 and 56 hours. After this period of time, both the cells and the supernant were collected, since the Ad could be found both within the cell nucleus and in the medium. Then 3 freeze/thaw cycles were conducted to release all the virus retained in the cells, and it was titrated by limiting dilution to find out the number of infectious units per microlitre in a 24-well plate with an estimated population of 50,000 cells per well. Once the total number of infectious units is known, it is possible to determine the number of infectious units generated per cell.

**7.1 Replication level of *helper* Ad and controls**

As Fig. 11 shows, at 36 hours the *helper* Ad Ad5/FC31 had produced hardly any viral particles, while the controls Ad5/attP and Ad5/GFP produced normal levels of virus. This fact had already been observed with the cell cycle, which reaffirmed that the *helper* Ad is incapable of packaging at 36 hours. However, at 56 hours the *helper* Ad could produce levels similar to the control Ads, providing evidence that its cell cycle is slower (56 hours) and that it can be amplified with enough production time.

**7.2 *Helper* Ad production percentage compared to controls.**

Table 3 shows the production percentage of Ad5/loxP.FC31 adenovirus compared to the controls, Ad5/attP and Ad5/GFP, at 36 hours in HEK293 cells. The data are as follows:

**Table 3.- Ad5/FC31 percentage compared to controls**

| % production in HEK293 (36 hours) | Ad5/loxP.FC31 / Ad5/attP | Ad5/loxP.FC31 / Ad5/GFP |
|---|---|---|
| | 0.0712% | 0.00133% |

As shown in Table 3, production levels indicate that Ad5/loxP.FC31 production is 1403 times less than Ad5/attP and 280.8.68 times less than Ad5/GFP at 36 hours in HEK293. Looking at Fig. 11, it can be concluded that after 56 hours the *helper* Ad produces a number of infectious units similar to the Ad control at 36 hours, showing that this *helper* Ad can produce viral particles within the timeframe of its viral cycle. If the *helper* Ad had a faster viral cycle, the production percentage would surely increase compared to its controls. However, due to the cell cycle delay for *helper* Ad compared to Ad5/GFP, the percentages are very low (0.01-0.001%), depending on the *helper* adenovirus used, showing the great potential of this system for the production of *helper*-free *gutless* Ad, especially when compared with current systems, which demonstrate percentages in the range of 0.1-10%. This implies that the invention's method would allow a 100- to 10,000-fold reduction in current *helper* virus contamination levels in *gutless* vector preparations.

### 7.3 Production percentage of helper Ad compared to controls

To finally understand the potential, not of purity or contamination, but rather its capability to act as "*helper*" for the invention's vector, it must be determined whether the viral cycle delay compared to the controls could be corrected with the help of proteins in trans supplied by a second control adenovirus (which would imply the low potential of the invention's vector). If, on the contrary, the genome is not packaged, regardless of whether the invention's *helper* adenovirus was exposed to proteins in trans, this would imply that the invention's method would still have high potential.

To accomplish this, experiments were conducted that involved cotransduction in 293 cells, which were infected with different combinations of virus at MOI=1 (AdBgal as control, and Ad5/IoxP.FC31 and Ad5/attP as *helpers*). The degree of infectivity of the adenovirus produced was analyzed at 36 hours.

As Fig. 12 shows, the cells were infected by a single adenovirus, and the production of the control vectors Ad5/attP and Ad5/Bgal were 100 times greater than those of Ad5/IoxP.FC31, When the cells were cotransduced by Ad5/attP + Ad5/Bgal, or Ad5/IoxP.FC31 + Ad5/Bgal, neither the Ad5/Bgal control levels (IU-Bgal) nor the levels of the helper Adenoviruses tested, Ad5/IoxP.FC31 and Ad5/attP (IU-GFP), were modified in comparison to the individual infections. Therefore, the altered packaging values were not recovered in the presence of viral proteins normally supplied in trans by control adenoviruses. This implies that the use of the attB and $\phi$C31 sequences is a system has great potential to produce *gutless* Ad that is essentially free of *helper* adenovirus.

### 7.4. Ability to act as *helper* to facilitate the multiplication and packaging of a *gutless* adenovirus

To confirm the ability of the invention's adenoviruses to act as *helpers*, an experiment was then conducted in triplicate involving the cotransduction of a plasmid that included the genome of a *gutless* adenovirus (pFK6, which contained a coding sequence for $\beta$-galactosidase and ITR and the $\Psi$ packaging signal for Ad5), with the plasmid for the Ad5/FC31.Cre adenovirus, PkP1.4$\Delta$CMV$\Delta$MCSattBIoxP$\Psi$GFPIoxPattP. Transduction took place in an equimolar ratio (3 micrograms of each of the plasmids per million cells) in HEK293 cells. The analysis of the infectious units produced from the transduction during the course of 36-40 hours showed that viral particles from *gutless* adenovirus were obtained, with an average value of 1577 IU//$10^6$ cell. (infectious units detected per each $10^6$ cells viewed), but *helper* adenovirus infectious particles were not detected, demonstrating that the sequences including the invention's *helper* adenovirus genome, with an attB sequence between the packaging signal $\Psi$ and the nearest ITR, are capable of provide all the mechanism needed for packaging the *gutless* adenovirus, even though its own packaging is delayed. The results obtained in each experiment, and the average value, are shown in Table 4.

**Table 4 .- Production of *gutless* and helper adenovirus in an experiment of cotransduction**

| | IU/$10^6$ cell | | | |
|---|---|---|---|---|
| | Experiment 1 | Experiment 2 | Experiment 3 | Average value |
| **PFK6** | 400 | 3200 | 1130 | 1577 |
| **Ad5/FC31.Cre** | 0 | 0 | 0 | 0 |

In summary, all these data show the enormous potential of using *helper* adenoviruses containing an attB sequence between the packaging signal and the closest ITR sequence to produce *gutless* adenovirus, making it feasible for the first time to think about *helper* adenovirus contamination levels sufficiently low (0.01-0001%) to allow its use in clinical trials in humans.

### REFERENCES

1 Alba R, Bosch A, Chillon M. Helper Dependent Adenovirus: Last improvements in gene therapy. Gene Ther 2005 (in press).

2 Mizuguchi H, Hayakawa T. Targeted adenovirus vectors. Hum Gene Ther 2004; 15: 1034-1044.

3 Horwitz MS. Adenoviridae and their replication. Virology 1990: 1679-1720.

4 Danthinne X, Imperiale MJ. Production of first generation adenovirus vectors: a review. Gene Ther 2000; 7: 1707-1714.
5 Benihoud K, Yeh P, Perricaudet M. Adenovirus vectors for gene delivery. Curr Opin Biotechnol 1999; 10: 440-447.
6 Gilgenkrantz H et al. Transient expression of genes transferred in vivo into heart using first-generation adenoviral vectors: role of the immune response. Hum Gene Ther 1995; 6: 1265-1274.
7 Yang Y, Haecker SE, Su Q, Wilson JM. Immunology of gene therapy with adenoviral vectors in mouse skeletal muscle. Hum Mol Genet 1996; 5: 1703-1712.
8 Mitani K, Graham FL, Caskey CT, Kochanek S. Rescue, propagation, and partial purification of a helper virus-dependent adenovirus vector. Proc Natl Acad Sci U S A 1995; 92: 3854-3858.
9 Parks RJ et al. A helper-dependent adenovirus vector system: removal of helper virus by Cre-mediated excision of the viral packaging signal. Proc Natl Acad Sci U S A 1996; 93: 13565-13570.
10 Palmer D, Ng P. Improved system for helper-dependent adenoviral vector production. Mol Ther 2003; 8: 846-852.
11 Sato M, Suzuki S, Kubo S, Mitani K. Replication and packaging of helper-dependent adenoviral vectors. Gene Ther 2002; 9: 472-476.
12 Soudais C, Skander N, Kremer EJ. Long-term in vivo transduction of neurons throughout the rat CNS using novel helper-dependent CAV-2 vectors. Faseb J 2004; 18: 391-393.
13 Kubo S, Saeki Y, Chiocca EA, Mitani K. An HSV amplicon-based helper system for helper-dependent adenoviral vectors. Biochem Biophys Res Commun 2003; 307: 826-830.
14 Cheshenko N, Krougliak N, Eisensmith RC, Krougliak VA. A novel system for the production of fully deleted adenovirus vectors that does not require helper adenovirus. Gene Ther 2001; 8: 846-854.
15 Kubo S, Mitani K. A new hybrid system capable of efficient lentiviral vector production and stable gene transfer mediated by a single helper-dependent adenoviral vector. J Virol 2003; 77: 2964-2971.
16 Schiedner G, Hertel S, Kochanek S. Efficient transformation of primary human amniocytes by E1 functions of Ad5: generation of new cell lines for adenoviral vector production. Hum Gene Ther 2000; 11: 2105-2116.
17 Fallaux FJ et al. New helper cells and matched early region 1-deleted adenovirus vectors prevent generation of replication-competent adenoviruses. Hum Gene Ther 1998; 9: 1909-1917.
18 Graham FL. Growth of 293 cells in suspension culture. J Gen Virol 1987; 68 (Pt 3): 937-940.
19 Sakhuja K et al. Optimization of the generation and propagation of gutless adenoviral vectors. Hum Gene Ther 2003; 14: 243-254
20 Thorpe HM, Wilson SE, Smith MC. Control of directionality in the site-specific recombination system of the Streptomyces phage phiC31. Mol Microbiol 2000; 38: 232-241.
21 Groth AC, Olivares EC, Thyagarajan B, Calos MP. A phage integrase directs efficient site-specific integration in human cells. Proc Natl Acad Sci U S A 2000; 97: 5995-6000.
22 Andreas S et al. Enhanced efficiency through nuclear localization signal fusion on phage PhiC31-integrase: activity comparison with Cre and FLPe recombinase in mammalian cells. Nucleic Acids Res 2002; 30: 2299-2306.
23 Alba R. MM, Rodriguez E., Gil E., Calos M.P, Bosch F., Chillón M. Generation of gutless helper free adenovirus using unidirectional FC31 recombinase as gene therapy vectors. Gene Ther 2004; 11: 164.
24 Ehrhardt A, Kay MA. A new adenoviral helper-dependent vector results in long-term therapeutic levels of human coagulation factor IX at low doses in vivo. Blood 2002; 99: 3923-3930.
25 Mian A et al. Long-term correction of ornithine transcarbamylase deficiency by WPRE-mediated overexpression using a helper-dependent adenovirus. Mol Ther 2004; 10: 492-499.
26 Gilbert R et al. Improved performance of a fully gutted adenovirus vector containing two full-length dystrophin cDNAs regulated by a strong promoter. Mol Ther 2002; 6: 501-509.
27 Zou L, Zhou H, Pastore L, Yang K. Prolonged transgene expression mediated by a helper-dependent adenoviral vector (hdAd) in the central nervous system. Mol Ther 2000; 2: 105-113.
28 Oshima Y et al. Intraocular gutless adenoviral-vectored VEGF stimulates anterior segment but not retinal neovascularization. J Cell Physiol 2004; 199: 399-411.
29 Koehler DR et al. Protection of Cftr knockout mice from acute lung infection by a helper-dependent adenoviral vector expressing Cftr in airway epithelia. Proc Natl Acad Sci U S A 2003; 100: 15364-15369.
30 Bilbao R et al. Comparison of high-capacity and first-generation adenoviral vector gene delivery to murine muscle in utero. Gene Ther 2005; 12: 39-47.
31 Ostapchuk P y Hearing P. Minimal cis-acting elements required for adenovirus genome packinging. J. Virol. 77: 5127-5135.
32 Thorpe et al. In vitro site-specific integration of bacteriophage DNA catalyzed by a recombinase of the resolvase/invertase family. Proc Natl Acad Sci U S A 1998, 5:5505-5510),
33 Hoess RH, Abremski K. Interaction of the bacteriophage P1 Recombinase Cre with the recognition site loxP. Proc Natl Acad Sci U S A 1984, 81(4):1026-1029.
34 Boussif O., Lezoualc'h F., Zanta M.A., Mergny M.D., Scherman D., Demeneix B., Behr J.P. A versatile vector for gene and oligonucleotide transfer into cells in culture and in vivo: polyethylenimine. Proc Natl Acad Sci U S A 1995, 92(16):7297-7301
35 Hearing P, Samulski RJ, Wishart WL, Shenk T. Identification of a repeated sequence element required for efficient encapsidation of the adenovirus type 5 chromosome. J Virol 1987; 61: 2555-2558.
36 Loonstra A et al. Growth inhibition and DNA damage induced by Cre recombinase in mammalian cells. Proc Natl Acad Sci U S A 2001; 98: 9209-9214.

**Claims**

1. Method for the production of gutless adenoviruses essentially free of helper adenovirus comprising the steps of:

   a) cotransducing the gutless adenovirus genome together with the helper adenovirus genome which includes a sequence $\phi$C31 attB sequence located 5' to the packaging signal $\Psi$, in a permissive cell line in which the expression of helper adenovirus proteins and the replication of the genomes of both adenoviruses is feasible;
   b) cultivating the cells under conditions where the expression of helper adenovirus proteins and the replication of both adenovirus genomes is possible;
   c) recovering the gutless adenovirus from the cell culture at time period after cotransfection that is greater than that needed for the gutless adenovirus to be packaged and less than that needed for the helper adenovirus to complete its viral cycle.

2. Method according to claim 1, comprising an additional previous step wherein the duration of the helper adenovirus cycle and the duration of the gutless adenovirus cycle in the cell line and the growing conditions to be used to carry out the multiplication of gutless adenovirus is determined.

3. Method according to any one of the claims 1 or 2, wherein the gutless adenovirus is recovered from the cell culture before the end of its viral cycle.

4. Method according to claim 3, wherein the gutless adenovirus is recovered from the cells in which it has multiplied itself, after removing the cells from the culture medium.

5. Method according to any one of claims 1 or 2, wherein the gutless adenovirus is recovered from the cell culture after it has completed its viral cycle.

6. Method according to claim 5, wherein the gutless adenovirus is recovered from the cell culture medium.

7. Method according to any one of the claims 1 to 6, wherein the cotransduction of the helper adenovirus genome occurs through its introduction into a cell as a part of a complete adenovirus particle.

8. Method according to claim 7, wherein the cotransduction of the gutless adenovirus genome occurs through its introduction into a cell without being part of a complete adenovirus particle.

9. Method according to claim 8, wherein the penetration of the gutless adenovirus genome into the cell without being part of a complete adenovirus particle is facilitated by the addition of calcium phosphate, lipofectamine, or PEI to the culture medium.

10. Method according to claim 7, wherein the cotransduction of the gutless adenovirus genome occurs through its introduction into a cell as a part of a complete adenovirus particle.

11. Method according to any one of the claims 1 through 6, wherein the cotransduction of the helper adenovirus genome occurs through its introduction into a cell without being part of a complete adenovirus particle.

12. Method according to claim 11, wherein the cotransduction of the gutless adenovirus genome occurs through its introduction into a cell without being part of a complete adenovirus particle.

13. Method according to any one of claims 11 to 12, wherein the introduction of the helper adenovirus genome into the cell and/or the introduction of the gutless adenovirus genome into the cell is facilitated by the addition of calcium phosphate, lipofectamine, or PEI to the culture medium.

14. Method according to claim 11, wherein the cotransduction of the gutless adenovirus genome occurs through its introduction into a cell as a part of a complete adenovirus particle.

15. Method according to any one of the claims 1 to 6, wherein the $\phi$C31 attB sequence located 5' to the packaging signal on the helper adenovirus genome comprises SEQ ID NO:2.

16. Method according to claim 15, wherein the helper adenovirus genome is the genome of a chimera adenovirus.

**17.** Method according to claim 16, wherein the sequences of the helper adenovirus genome derived from adenovirus genomic sequences derive from human serotype 5 except for the coding sequence corresponding to the fibre protein.

**18.** Method according to claim 17, wherein the coding sequence corresponding to the fibre protein derives from human serotype 40.

**19.** Method according to claim 17, wherein the sequences of the helper adenovirus genome derived from adenovirus genome sequences derive from human serotype 5 except for the coding sequence corresponding to the fibre protein and the coding sequence corresponding to the protein that forms the penton.

**20.** Method according to claim 16, wherein the chimera helper adenovirus genome comprises sequences derived from at least one nonhuman serotype and sequences derived from at least one human serotype.

**21.** Method according to claim 20, wherein the sequences derived from nonhuman serotypes derive from canine serotype CAV-2.

**22.** Method according to any one of claims 16 to 21, wherein the helper adenovirus genome lacks the sequences corresponding to the E1 region.

**23.** Method according to claim 15, wherein the helper adenovirus genome sequences derived from adenovirus genome sequences are all derived from a single serotype, isolated from any species.

**24.** Method according to claim 23, wherein the helper adenovirus genome sequences derived from adenovirus genome sequences are all derived from human serotype 5.

**25.** Method according to claim 24, wherein the helper adenovirus genome lacks the sequences that corresponding to the E1 region.

**26.** Method according to claim 25, wherein the helper adenovirus genome also lacks the sequences corresponding to the E3 region.

**27.** Method according to claims 25 or 26, wherein the attB sequence is represented by SEQ ID NO:2.

**28.** Method according to claim 27, wherein the attB sequence is inserted at nucleotide 193 of the sequence that corresponds to the human serotype 5 adenovirus genome.

**29.** Method according to any one of claims 25 to 28, wherein the helper adenovirus genome also comprises an attP sequence located 3' to the packaging signal.

**30.** Method according to claim 29, wherein the attP sequence present in the helper adenovirus genome comprises SEQ ID NO:3.

**31.** Method according to claim 30, wherein the attP sequence present in the helper adenovirus genome is represented by SEQ ID NO:4.

**32.** Method according to any one of claims 29 to 31, wherein the attP sequence present in the helper adenovirus genome is found at a distance of 1.5 - 2 kb from the attB the sequence

**33.** Method according to claim 32, wherein the helper adenovirus genome corresponds to that of the Ad5/FC31 adenovirus.

**34.** Method according to any one of claims 29 to 32, wherein the helper adenovirus genome also comprises a loxP sequence located 5' to the packaging signal Ψ.

**35.** Method according to claim 34, wherein the loxP sequence located 5' to the packaging signal Ψ is located between the attB sequence and the packaging signal Ψ.

**36.** Method according to claim 35, wherein the helper adenovirus genome comprises the sequence represented by SEQ ID NO:15 in its 5' region.

**37.** Method according to claim 36, wherein the helper adenovirus genome corresponds to that of the Ad5/loxP.FC31 adenovirus

**38.** Method according to any one of claims 34 or 35, wherein the helper adenovirus genome also comprises a loxP sequence located 3' to the packaging signal Ψ.

**39.** Method according to claim 38, wherein the loxP sequence located 3' to the packaging signal Ψ is located between the attP sequence and the packaging signal Ψ.

**40.** Method according to claim 39, wherein the helper adenovirus genome corresponds to that of the Ad5/FC31.Cre adenovirus

**41.** Method according to any one of claims 25 to 28, wherein the helper adenovirus genome also comprises at least one loxP sequence located 5' to the packaging signal Ψ and at least one loxP sequence located 3' to the packaging signal Ψ.

**42.** Method according to claim 41, wherein the loxP sequence located 5' to the packaging signal Ψ is located between the attB sequence and the packaging signal Ψ and the loxP sequence located 3' to the packaging signal is located between the attP sequence and the packaging signal Ψ.

**43.** Method according to claim 42, wherein the helper adenovirus genome corresponds to that of the Ad5/attB.Cre adenovirus.

**44.** Method according to any one of claims 22 or 25 to 43, wherein the cotransduction of the gutless adenovirus genome together with the helper adenovirus genome occurs in a permissive cell line capable of supplying in trans proteins able to complement the function corresponding to the proteins of the helper adenovirus E1 region.

**45.** Method according to claim 44, wherein the cotransduction of the gutless adenovirus genome together with the helper adenovirus genome occurs in a cell line which is selected from HEK293, PERC6, and N52E6.

**46.** Method according to claim 45, wherein the cotransduction of the gutless adenovirus together with the helper adenovirus occurs in the cell line HEK293.

**47.** Method according to any one of claims 44 to 46, wherein the gutless adenovirus is recovered from the cells in which it has multiplied itself after removing the cells from the culture medium.

**48.** Method according to claim 47, wherein the gutless adenovirus is recovered from the cell culture less than 36 hours after cotransfection.

**49.** Method according to any one of claims 44 to 46, wherein the gutless adenovirus is recovered from the cell culture after having completed its viral cycle.

**50.** Method according to claim 49, wherein the gutless adenovirus is recovered from the cell culture more than 36 hours after cotransfection.

**51.** Method according to claim 50, wherein the gutless adenovirus is recovered from the cell culture after less than 56 hours after cotransfection.

**52.** Method according to any one of the claims 44 through 51, wherein the helper adenovirus genome penetrates the cell without being part of a complete adenovirus particle.

**53.** Method according to any one of claims 44 to 51, wherein the nucleotide sequence that comprises the helper adenovirus genome penetrates the cell as a part of a complete adenovirus particle.

**54.** Method according to any one of claims 52 or 53, wherein the nucleotide sequence that constitutes the helper adenovirus genome is selected from among those of the Ad5/loxP.FC31, Ad5/FC31.Cre, Ad5/FC31, and Ad5/attB.Cre adenovirus genomes.

**55.** Method according to claim 23, wherein the sequences of the helper adenovirus genome derived from adenovirus genome sequences are derived from a nonhuman serotype.

**56.** Method according to claim 55, wherein the helper adenovirus genome sequences derive from canine serotype CAV-2.

**57.** Method according to any one of claims 1 to 15, wherein the cotransduction of the gutless adenovirus genome together with the helper adenovirus genome is produced in a permissive cell line that expresses at least one recombinase functional in eukaryotic cells.

**58.** Method according to claim 57, wherein the cotransduction of the gutless adenovirus genome together with the helper adenovirus genome is produced in a permissive cell line that expresses at least one functional recombinase in eukaryotic cells that is selected from among the ϕC31 recombinase, the Cre recombinase, or the FLPe recombinase.

**59.** Method according to claim 58, wherein the cotransduction of the gutless adenovirus genome together with the helper adenovirus genome occurs in a permissive cell line that expresses at least the ϕC31 recombinase.

**60.** Method according to claim 59, wherein the cell line in which the cotransduction of the gutless adenovirus genome together with the helper adenovirus genome occurs is 293ϕC31.

**61.** Method according to any one of claims 59 or 60, wherein the helper adenovirus genome comprises, additionally to the attB sequence, an ϕ31 attP sequence located 3' to the packaging signal.

**62.** Method according to claim 58 wherein the cotransduction of the gutless adenovirus genome together with the helper adenovirus genome occurs in a permissive cell line that expresses at least the Cre recombinase.

**63.** Method according to claim 62, wherein the cell line in which the cotransduction of the gutless adenovirus genome together with the helper adenovirus genome occurs is 293Cre.

**64.** Method according to any one of claims 62 or 63, wherein the helper adenovirus genome also comprises at least two loxP sequences, one located 5' to the packaging signal $\Psi$ and the other one located 3' to the packaging signal $\Psi$.

**65.** Method according to claim 58, wherein the cotransduction of the gutless adenovirus together with the helper adenovirus occurs in a permissive cell line that expresses at least the FLPe recombinase.

**66.** Method according to claim 65, wherein the cell line in which the cotransduction of the gutless adenovirus genome together with the helper adenovirus genome occurs, is 293FLPe.

**67.** Method according to any one of claims 65 or 66, wherein the helper adenovirus comprises at least two frt sequences, one located 5' to the packaging signal $\Psi$ and the other one located 3' to the packaging signal $\Psi$.

**68.** Method according to claim 57, wherein the cotransduction of the gutless adenovirus together with the helper adenovirus occurs in a permissive cell line that expresses at least two recombinases functional in eukaryotic cells

**69.** Method according to claim 68, wherein the cotransduction of the gutless adenovirus genome together with the helper adenovirus genome occurs in a permissive cell line that expresses at least two recombinases that are functional in eukaryotic cells, that is selected from among the ϕC31 recombinase, the Cre recombinase, or the FLPe recombinase.

**70.** Method according to claim 69, wherein the cotransduction of the gutless adenovirus genome together with the helper adenovirus genome occurs in a permissive cell line that expresses at least the ϕC31 recombinase and the Cre recombinase

**71.** Method according to claim 70, wherein the cell line in which the cotransduction of the gutless adenovirus genome together with the helper adenovirus genome occurs, is 293CreϕC31.

**72.** Method according to any one of claims 70 or 71, wherein the helper adenovirus comprises, additionally to the attB sequence, a ϕC31 attP sequence located 3' to the packaging signal and at least two loxP sequences, one located 5' to the packaging signal Ψ and the other one located 3' to the packaging signal Ψ.

**73.** Method according to any one of the precedent claims, wherein the ITR and the packaging signal Ψ present in the adenovirus gutless genome derive from the same adenovirus serotype as the ITR and the packaging signal Ψ present in the helper adenovirus genome that is cotransduced together with the gutless adenovirus genome.

**74.** A polynucleotide sequence that comprises, at its ends, adenovirus ITRs separated by a distance that allows its packaging within an adenovirus particle, a packaging signal Ψ closer to one of the ITR that to the other and separated from the first ITR by a distance that does not prevent the packaging of the sequence inside an adenovirus particle, sequences coding for adenovirus proteins controlled by adenovirus promoters located between the packaging signal Ψ and the ITR farthest from it, all in an arrangement similar to that of adenovirus genomes, and that additionally comprises a ϕC3 attB sequence located 5' to the packaging signal Ψ.

**75.** Sequence according to claim 74, wherein the ϕC31 attB sequence located 5' to the packaging signal Ψ comprises SEQ ID NO:2.

**76.** Sequence according to claim 75, wherein the sequences derived from adenovirus genomes derive from at least two different adenovirus serotypes.

**77.** Sequence according to claim 76, wherein the sequences derived from adenovirus genomes are all derived from human serotype 5 except for the coding sequence that corresponding to the fibre protein.

**78.** Sequence according to claim 77, wherein the coding sequence corresponding to the fibre protein is derived from human serotype 40.

**79.** Sequence according to claim 76, wherein the sequences derived from adenovirus genomes are all derived from human serotype 5 except for the coding sequence corresponding to fibre protein and the coding sequence corresponding to the protein that forms the penton.

**80.** Sequence according to claim 75, wherein the sequences derived from adenovirus genomes are all derived from human serotype 5.

**81.** Sequence according to claim 80, which comprises all the sequences that belong to an adenovirus genome, with the exception of the sequences corresponding to the E1 region.

**82.** Sequence according to claim 80, which comprises all the sequences that belong to an adenovirus genome, with the exception of the sequences corresponding to the E1 region and the sequences corresponding to the E3 region.

**83.** Sequence according to claim 81 or 82, wherein the ϕC31 attB sequence located 5' to the packaging signal Ψ is represented by SEQ ID NO:2.

**84.** Sequence according to claim 83, wherein the attB sequence is inserted at nucleotide 193 of the sequence corresponding to the human serotype 5 adenovirus genome.

**85.** Sequence according to any one of claims 82 to 84, which also comprises an ϕC31 attP sequence located 3' to the packaging signal Ψ.

**86.** Sequence according to claim 85, wherein the attP sequence comprises the sequence represented by SEQ ID NO:3.

**87.** Sequence according to claim 86, wherein the attP sequence is that represented by SEQ ID NO:4.

**88.** Sequence according to claim 87, wherein the attB sequence is separated from the attP sequence by a distance of 1.5-2 kb.

**89.** Sequence according to claim 88, which comprises the Ad5/FC31 adenovirus genome.

**90.** Sequence according to claim 89, which matches Ad5/FC31 adenovirus genome sequence.

**91.** Sequence according to any one of claims 85 to 90, which is associated with adenoviral proteins forming a complete adenovirus particle.

**92.** Sequence according to any one of the claims 85 through 89, which is a part of a plasmid.

**93.** Sequence according to claim 92, which matches the PkP1.4ΔCMVΔMCSattBΔGFPattP plasmid sequence.

**94.** Sequence according to any one of claims 85 to 88, which also comprises a loxP sequence located 5' to the packaging signal Ψ.

**95.** Sequence according to claim 94, wherein the loxP sequence located 5' to the packaging signal Ψ is located between the attB sequence and the packaging signal Ψ.

**96.** Sequence according to claim 95, which comprises the Ad5/loxP.FC31 adenovirus genome.

**97.** Sequence according to claim 96, which matches the Ad5/loxP.FC31 adenovirus genome.

**98.** Sequence according to any one of claims 94 to 97, which is associated with adenoviral proteins forming a complete adenovirus particle.

**99.** Sequence according to any one of claims 94 to 96, which is part of a plasmid.

**100.** Sequence according to claim 99, which comprises the sequence represented by SEQ ID NO:16.

**101.** Sequence according to claim 100, which matches the PkP1.4ΔCMVΔMCSattBΔGFPattP plasmid sequence.

**102.** Sequence according to any one of claims 94 or 95, which also comprises a loxP sequence located 3' to the packaging signal Ψ.

**103.** Sequence according to claim 102, wherein the loxP sequence located 3' to the packaging signal Ψ is located between the packaging signal Ψ and the attP sequence.

**104.** Sequence according to claim 103, which comprises the Ad5/FC31.Cre adenovirus genome.

**105.** Sequence according to claim 104, which matches the Ad5/FC31.Cre adenovirus genome sequence.

**106.** Sequence according to any one of claims 102 to 104, which is associated with adenoviral proteins forming a complete adenovirus particle.

**107.** Sequence according to any one of claims 102 to 104, which is part of a plasmid.

**108.** Sequence according to claim 107, which matches the PkP1.4ΔCMVΔMCSattBΔGFPattP plasmid sequence.

**109.** Sequence according to any one of claims 81 to 84, which also comprises a loxP sequence located 5' to the packaging signal Ψ and a loxP sequence located 3' to the packaging signal Ψ.

**110.** Sequence according to claim 109, which comprises the Ad5/attB.Cre adenovirus genome.

**111.** Sequence according to claim 110, which matches the Ad5/attB.Cre adenovirus genome.

**112.** Sequence according to any one of claims 109 to 111, which is associated with adenoviral proteins forming a complete adenovirus particle.

**113.** Sequence according to any one of claims 109 or 110, which is part of a plasmid.

**114.** Sequence according to claim 113, which is the PkP1.4ΔCMVΔMCSattBΔGFPattP plasmid sequence.

**115.** Sequence according to claim 75, wherein the sequences derived from adenovirus genomes derive from adenoviruses of one or more serotypes, other than human serotype 5.

**116.** Sequence according to claim 115, wherein the sequences derived from adenovirus genomes are derived in part or in whole from canine serotype CAV-2.

**117.** Use of a sequence according to any one of claims 74 to 116 for the production of gutless adenovirus.

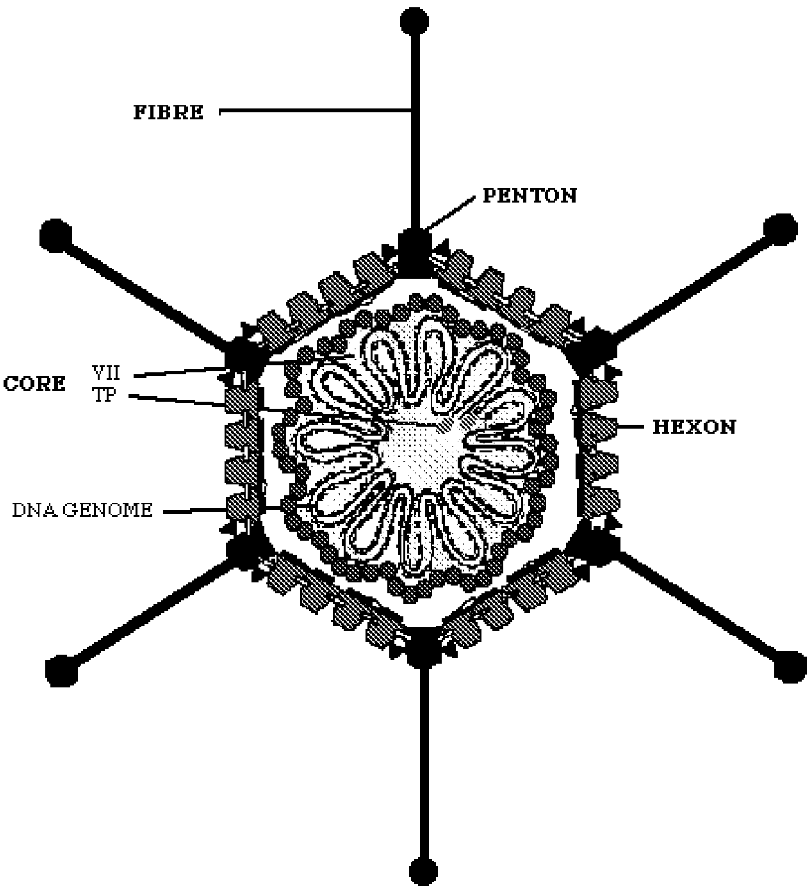


Fig. 1

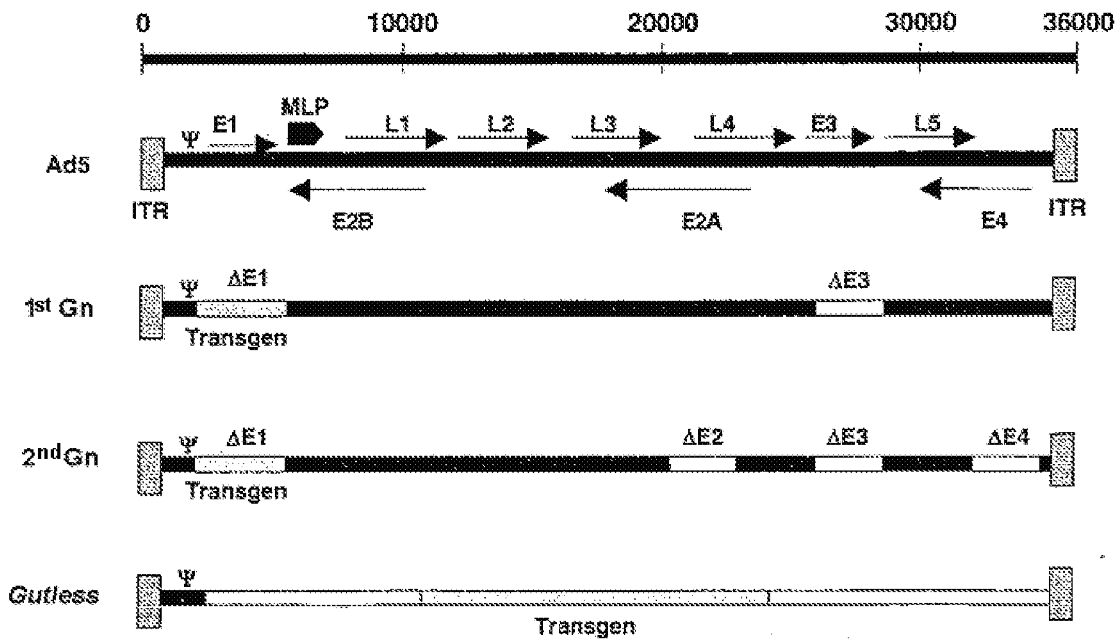
0
10000
20000
30000
36000
Ad5
ITR
Ψ
E1
MLP
L1
L2
L3
L4
E3
L5
ITR
E2B
E2A
E4
1st Gn
ΔE1
ΔE3
Transgen
2ndGn
ΔE1
ΔE2
ΔE3
ΔE4
Transgen
Gutless
Ψ
Transgen

Fig. 2

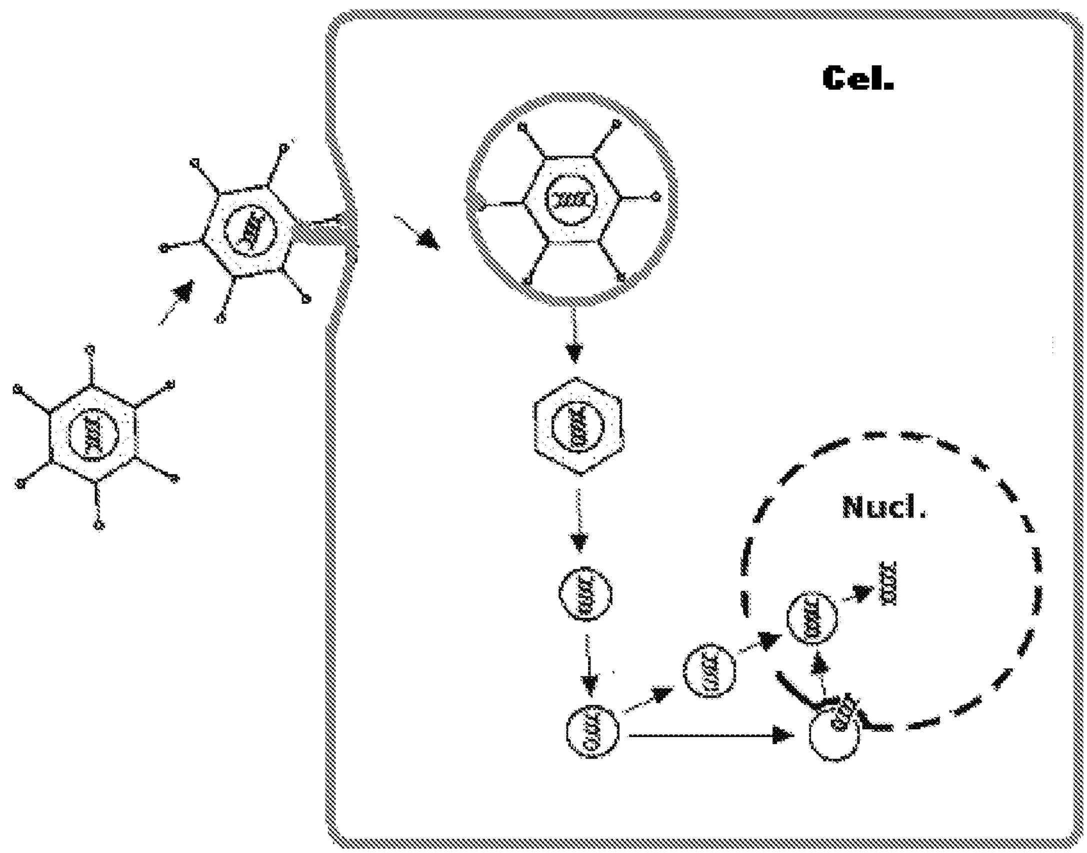


Fig. 3

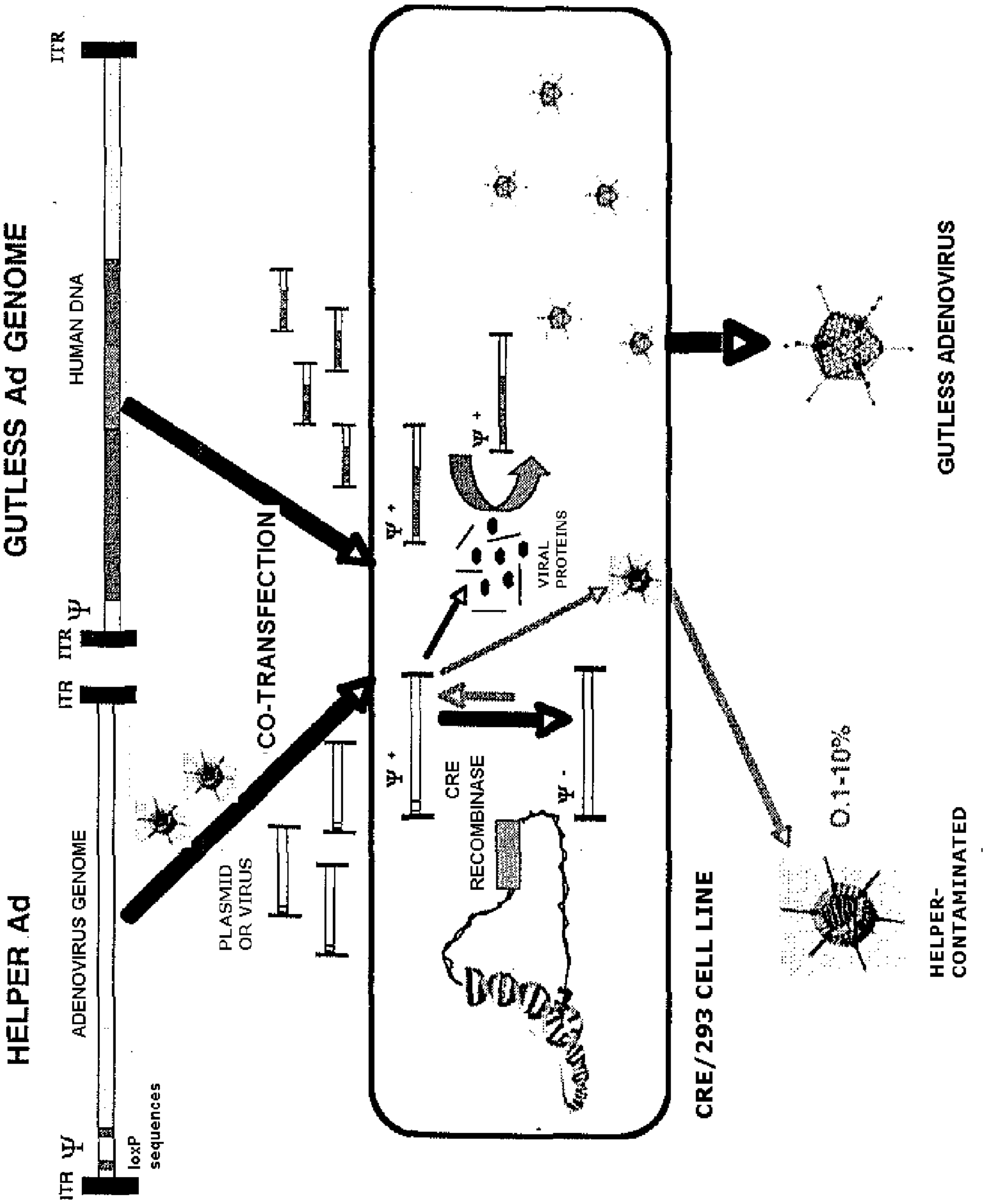
HELPER Ad
GUTLESS Ad GENOME
ITR
Ψ
ITR
Ψ
loxP sequences
ADENOVIRUS GENOME
ITR
ITR
Ψ
HUMAN DNA
CO-TRANSFECTION
PLASMID OR VIRUS
Ψ +
Ψ +
Ψ +
CRE RECOMBINASE
Ψ -
VIRAL PROTEINS
CRE/293 CELL LINE
0.1-10%
HELPER-CONTAMINATED
GUTLESS ADENOVIRUS

Fig. 4

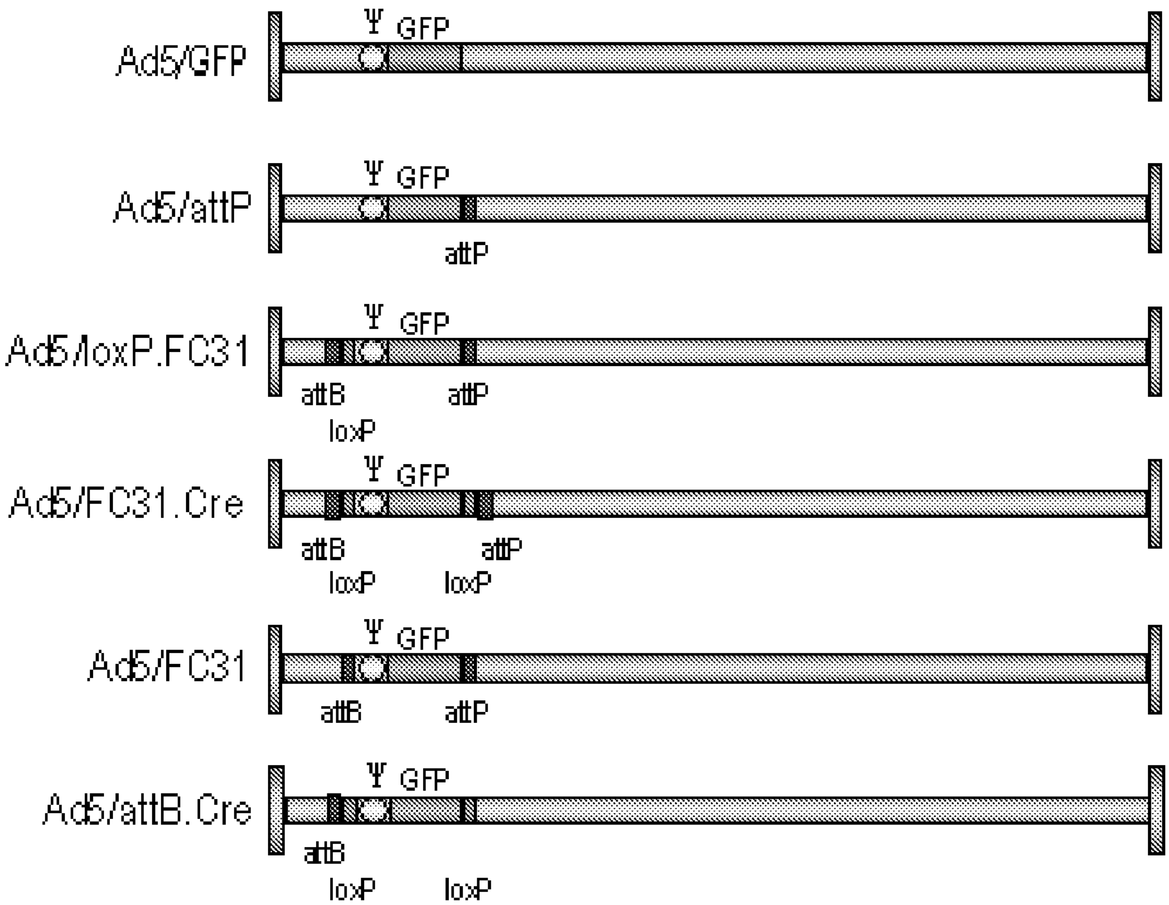
Ad5/GFP
Ψ GFP
Ad5/attP
Ψ GFP
attP
Ad5/loxP.FC31
Ψ GFP
attB
loxP
attP
Ad5/FC31.Cre
Ψ GFP
attB
loxP
attP
loxP
Ad5/FC31
Ψ GFP
attB
attP
Ad5/attB.Cre
Ψ GFP
attB
loxP
loxP

Fig. 5

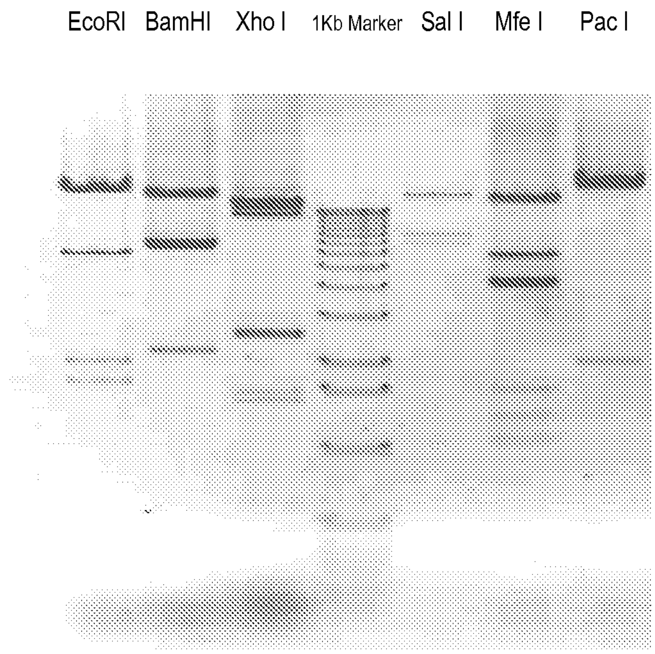


Fig. 6

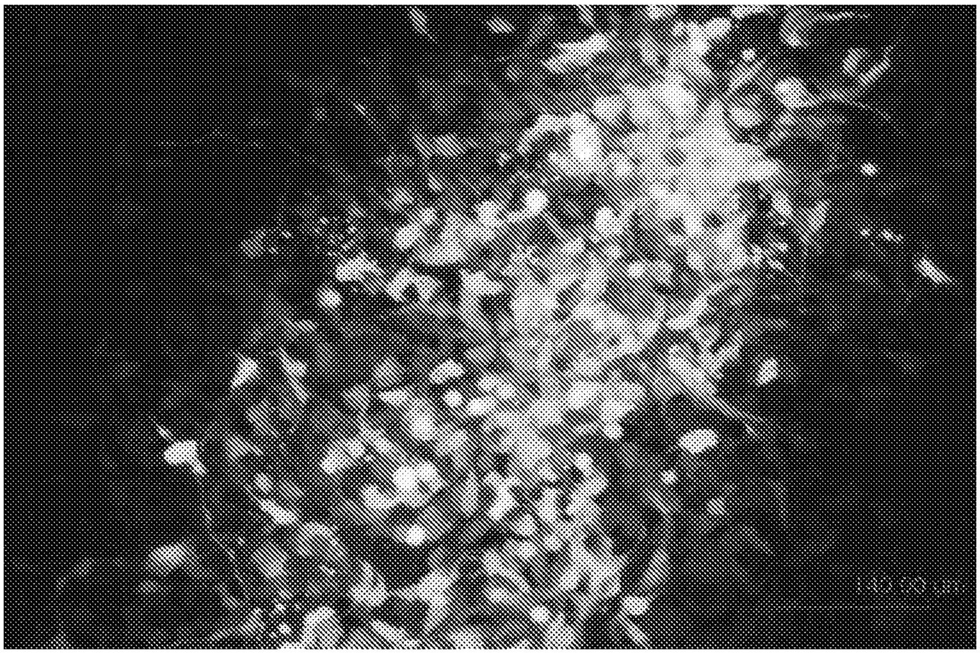

Fig. 7

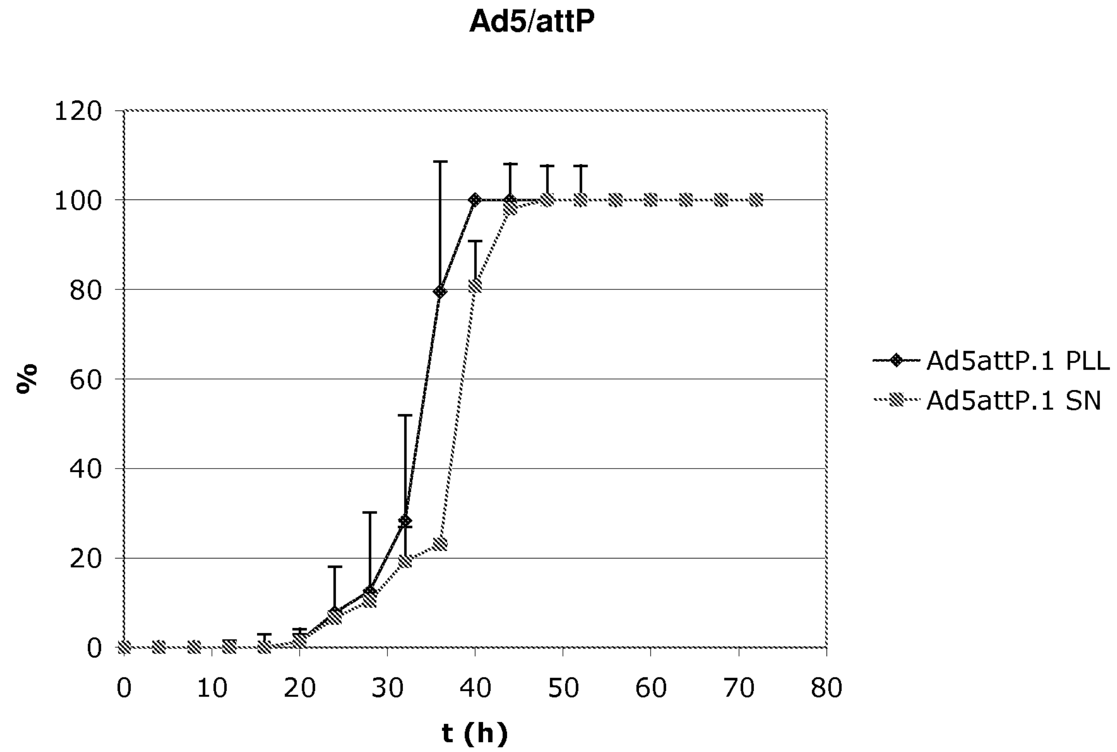
Ad5/attP
120
100
80
60
40
20
0
%
0
10
20
30
40
50
60
70
80
t (h)
Ad5attP.1 PLL
Ad5attP.1 SN

Fig. 8a

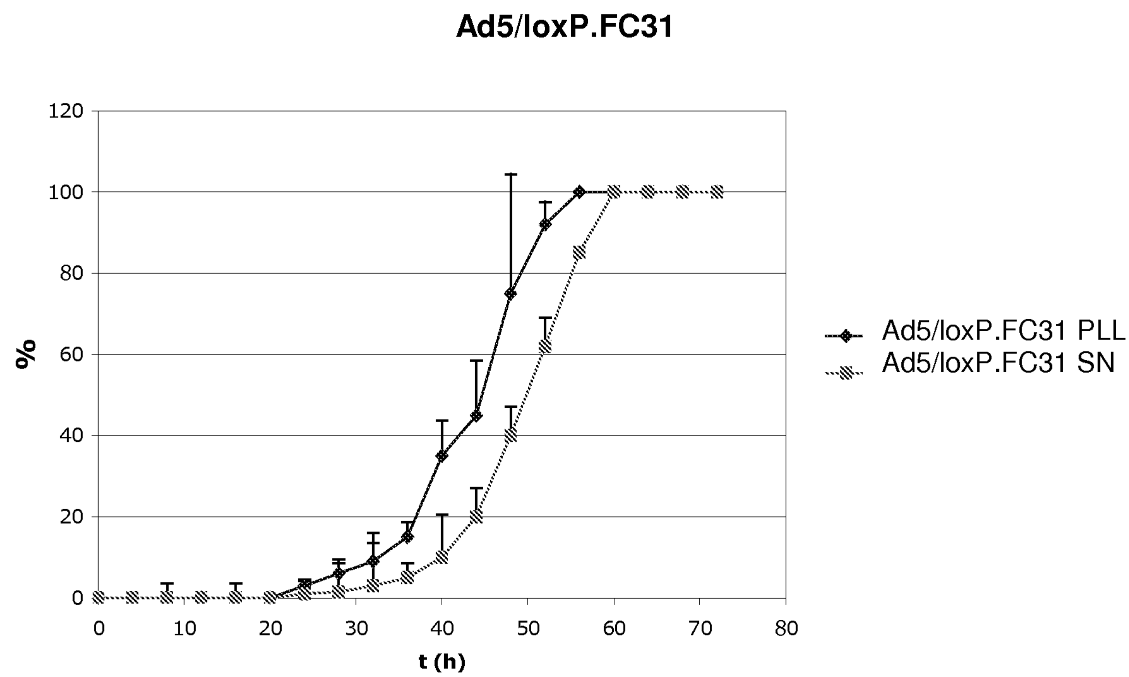
Ad5/loxP.FC31
120
100
80
60
40
20
0
%
0
10
20
30
40
50
60
70
80
t (h)
Ad5/loxP.FC31 PLL
Ad5/loxP.FC31 SN

Fig. 8b

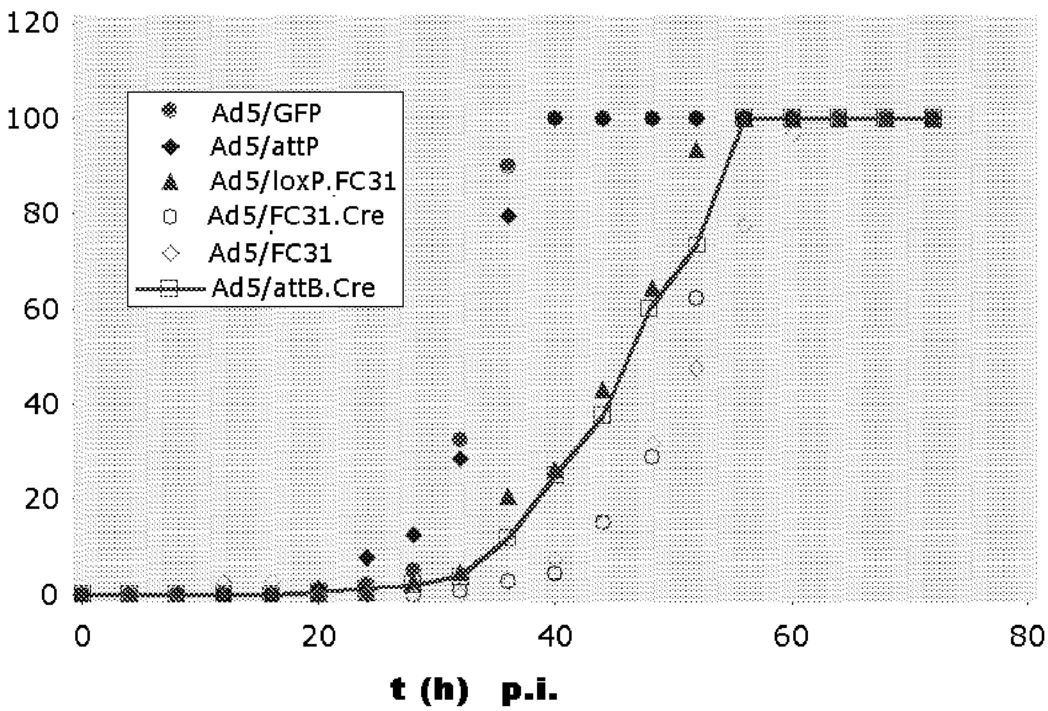
120
100
80
60
40
20
0
0
20
40
60
80
t (h) p.i.
Ad5/GFP
Ad5/attP
Ad5/loxP.FC31
Ad5/FC31.Cre
Ad5/FC31
Ad5/attB.Cre

Fig. 9

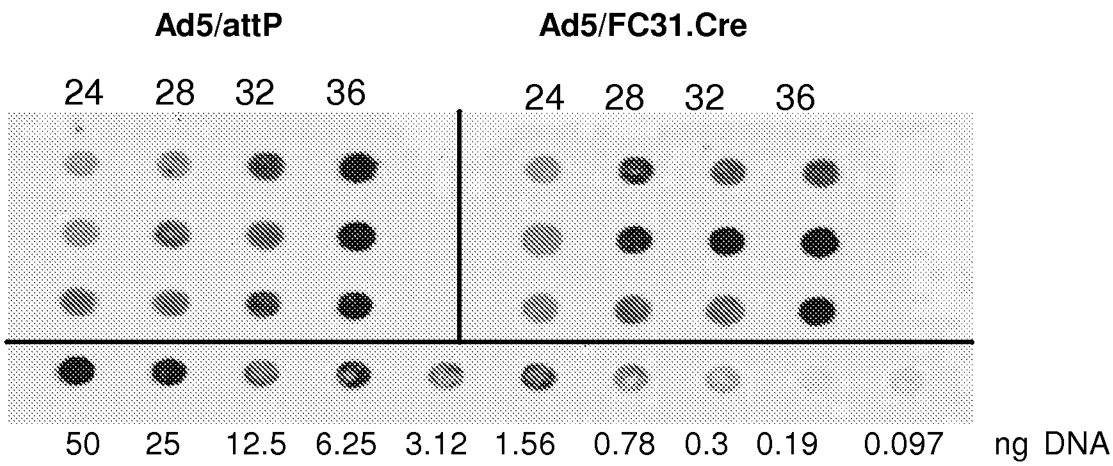
Ad5/attP
Ad5/FC31.Cre
24
28
32
36
24
28
32
36
50
25
12.5
6.25
3.12
1.56
0.78
0.3
0.19
0.097
ng DNA

Fig. 10

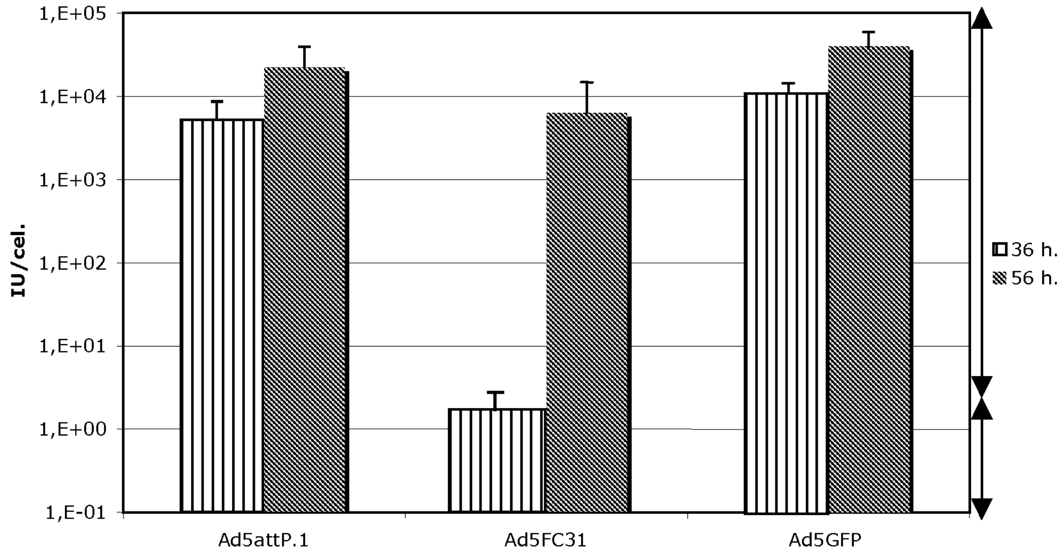
1,E+05
1,E+04
1,E+03
1,E+02
1,E+01
1,E+00
1,E-01
IU/cel.
36 h.
56 h.
Ad5attP.1
Ad5FC31
Ad5GFP

Fig. 11

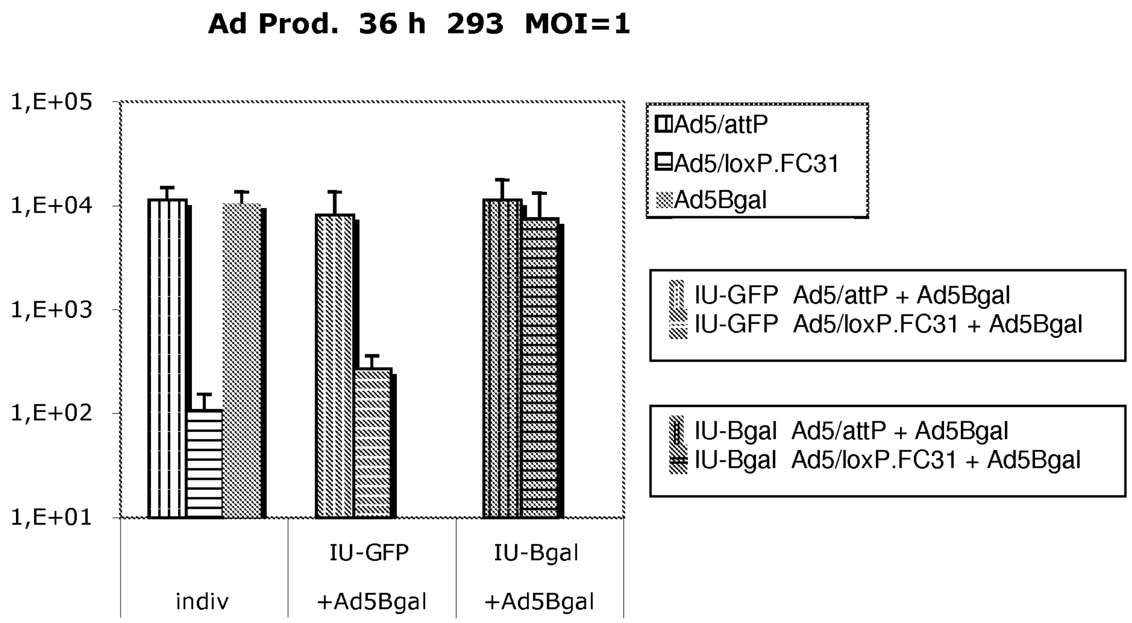
Ad Prod. 36 h 293 MOI=1
1,E+05
1,E+04
1,E+03
1,E+02
1,E+01
indiv
IU-GFP +Ad5Bgal
IU-Bgal +Ad5Bgal
Ad5/attP
Ad5/loxP.FC31
Ad5Bgal
IU-GFP Ad5/attP + Ad5Bgal
IU-GFP Ad5/loxP.FC31 + Ad5Bgal
IU-Bgal Ad5/attP + Ad5Bgal
IU-Bgal Ad5/loxP.FC31 + Ad5Bgal

Fig. 12

# INTERNATIONAL SEARCH REPORT

International application No.
PCT/ ES 2007/000260

A. CLASSIFICATION OF SUBJECT MATTER

see extra sheet

According to International Patent Classification (IPC) or to both national classification and IPC

B. FIELDS SEARCHED

Minimum documentation searched (classification system followed by classification symbols)
C12N

Documentation searched other than minimum documentation to the extent that such documents are included in the fields searched

Electronic data base consulted during the international search (name of data base and, where practicable, search terms used)

CIBEPAT, EPODOC, WPI, TXTE, TXTF, MEDLINE, BIOSIS, XPESP, EMBASE, NPL, EMBL ALL, NA GENESEQ

C. DOCUMENTS CONSIDERED TO BE RELEVANT

| Category* | Citation of document, with indication, where appropriate, of the relevant passages | Relevant to claim No. |
|---|---|---|
| X | WO 2001/021824 A1 (GENZYME CORPORATION) 29.03.2001, the whole document. | 74, 117 |
| Y | | 75-77, 79-88, 91, 92, 115, 116 |
| A | | 1-17, 19-32, 34-36, 38, 39, 41, 42, 44-53, 55-73, 94, 95, 98-100, 102, 103, 106, 107, 109, 112, 113 |
| Y | WO 2002/077246 A2 (ICON GENETICS AG; ICON GENETICS, INC.) 03.10.2002, examples 12, 13; figure 12. | 75-77, 79-88, 91, 92, 115, 116 |

☒ Further documents are listed in the continuation of Box C. ☒ See patent family annex.

* Special categories of cited documents:

"A" document defining the general state of the art which is not considered to be of particular relevance.

"E" earlier document but published on or after the international filing date

"L" document which may throw doubts on priority claim(s) or which is cited to establish the publication date of another citation or other special reason (as specified)

"O" document referring to an oral disclosure use, exhibition, or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other documents , such combination being obvious to a person skilled in the art

"&" document member of the same patent family

| | |
|---|---|
| Date of the actual completion of the international search<br>26 September 2007 (26.09.2007) | Date of mailing of the international search report<br>(01/10/2007) |
| Name and mailing address of the ISA/<br>O.E.P.M.<br>Paseo de la Castellana, 75 28071 Madrid, España.<br>Facsimile No. 34 91 3495304 | Authorized officer<br>E. Relaño Reyes<br>Telephone No. +34 91 349 85 04 |

Form PCT/ISA/210 (second sheet) (April 2007)

INTERNATIONAL SEARCH REPORT

International application No.
PCT/ES 2007/000260

C (continuation). DOCUMENTS CONSIDERED TO BE RELEVANT

| Category* | Citation of documents, with indication, where appropriate, of the relevant passages | Relevant to claim No. |
|---|---|---|
| X<br><br>A | ALBA, R., MONFAR, M., RODRÍGUEZ, E. et al. Generation of gutless helper free adenovirus using unidirectional ϕc31 recombinase as gene therapy vectors. Abstracts 2nd European Conference and Practical Course: Poster Communications 1-72. Gene Therapy. October 2004, Vol. 11, Supplement 1, page S146. ISSN 0969-7128. | 74, 117<br><br>1-17, 19-32, 34-36, 38, 39, 41, 42, 44-53, 55-73, 75-77, 79-88, 91, 92, 94, 95, 98-100, 102, 103, 106, 107, 109, 112, 113, 115, 116 |
| X<br><br>A | ALBA, R., RODRÍGUEZ, E., MAULEON, I. et al. Generación de adenovirus gutless *helper free* mediante el uso del doble sistema de recombinasas C31/CRE. III Reunión de la SETG, Pamplona 2005. [online][published on line the 09.02.2005], [retrieved the 24.09.2007]. Retrieved from the Internet: <URL: http://www.uv.es/ SETG/> | 74, 117<br><br>1-17, 19-32, 34-36, 38, 39, 41, 42, 44-53, 55-73, 75-77, 79-88, 91, 92, 94, 95, 98-100, 102, 103, 106, 107, 109, 112, 113, 115, 116 |
| X<br><br>A | ALBA, R., BOSCH, A., CHILLON, M. Gutless adenovirus: last-generation adenovirus for gene therapy. Gene Therapy. October 2005, Vol. 12, Suplemento 1, pages S18-S27. ISSN 0969-7128. | 74, 117<br><br>1-17, 19-32, 34-36, 38, 39, 41, 42, 44-53, 55-73, 75-77, 79-88, 91, 92, 94, 95, 98-100, 102, 103, 106, 107, 109, 112, 113, 115, 116 |
| A | WO 2000/046360 A1 (MERCK & CO., INC.) 10.08.2000, the whole document. | 1-17, 19-32, 34-36, 38, 39, 41, 42, 44-53, 55-73 |

Form PCT/ISA/210 (continuation of second sheet) (April 2007)

INTERNATIONAL SEARCH REPORT

Information on patent family members

International application No.

PCT/ ES 2007/000260

| Patent document cited in the search report | Publication date | Patent family member(s) | Publication date |
|---|---|---|---|
| WO 2001/021824 A1 | 29.03.2001 | CA 2388365 A<br>AU 7579100 A<br>US 6541245 B1 | 29.03.2001<br>24.04.2001<br>01.04.2003 |
| WO 2002/077246 A2 | 03.10.2002 | CA 2442254 A<br>DE 10114209 A1<br>EP 1392833 A2<br>JP 2004524039 T<br>US 2005066384 A1<br>AU 2002302460 B | 03.10.2002<br>05.12.2002<br>03.03.2004<br>12.08.2004<br>24.03.2005<br>30.08.2007 |
| WO 2000/046360 A1 | 10.08.2000 | CA 2360796 A<br>EP 1151091 A1<br>JP 2002535986 T | 10.08.2000<br>07.11.2001<br>29.10.2002 |

Form PCT/ISA/210 (patent family annex) (April 2007)

INTERNATIONAL SEARCH REPORT

International application No.

PCT/ ES 2007/000260

CLASSIFICATION OF SUBJECT MATTER

*C12N 7/02* (2006.01)
*C12N 15/861* (2006.01)

Form PCT/ISA/210 (extra sheeet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ALBA R ; BOSCH A ; CHILLON M.** Helper Dependent Adenovirus: Last improvements in gene therapy. *Gene Ther,* 2005
- **MIZUGUCHI H ; HAYAKAWA T.** Targeted adenovirus vectors. *Hum Gene Ther,* 2004, vol. 15, 1034-1044
- **HORWITZ MS.** Adenoviridae and their replication. *Virology,* 1990, 1679-1720
- **DANTHINNE X ; IMPERIALE MJ.** Production of first generation adenovirus vectors: a review. *Gene Ther,* 2000, vol. 7, 1707-1714
- **BENIHOUD K ; YEH P ; PERRICAUDET M.** Adenovirus vectors for gene delivery. *Curr Opin Biotechnol,* 1999, vol. 10, 440-447
- **GILGENKRANTZ H et al.** Transient expression of genes transferred in vivo into heart using first-generation adenoviral vectors: role of the immune response. *Hum Gene Ther,* 1995, vol. 6, 1265-1274
- **YANG Y ; HAECKER SE ; SU Q ; WILSON JM.** Immunology of gene therapy with adenoviral vectors in mouse skeletal muscle. *Hum Mol Genet,* 1996, vol. 5, 1703-1712
- **MITANI K ; GRAHAM F ; CASKEY CT ; KOCHANEK S.** Rescue, propagation, and partial purification of a helper virus-dependent adenovirus vector. *Proc Natl Acad Sci U S A,* 1995, vol. 92, 3854-3858
- **PARKS RJ et al.** A helper-dependent adenovirus vector system: removal of helper virus by Cre-mediated excision of the viral packaging signal. *Proc Natl Acad Sci U S A,* 1996, vol. 93, 13565-13570
- **PALMER D, NG P.** Improved system for helper-dependent adenoviral vector production. *Mol Ther,* 2003, vol. 8, 846-852
- **SATO M ; SUZUKI S ; KUBO S ; MITANI K.** Replication and packaging of helper-dependent adenoviral vectors. *Gene Ther,* 2002, vol. 9, 472-476
- **SOUDAIS C ; SKANDER N ; KREMER EJ.** Long-term in vivo transduction of neurons throughout the rat CNS using novel helper-dependent CAV-2 vectors. *Faseb J,* 2004, vol. 18, 391-393
- **KUBO S ; SAEKI Y ; CHIOCCA EA ; MITANI K.** An HSV amplicon-based helper system for helper-dependent adenoviral vectors. *Biochem Biophys Res Commun,* 2003, vol. 307, 826-830
- **CHESHENKO N ; KROUGLIAK N ; EISENSMITH RC ; KROUGLIAK VA.** A novel system for the production of fully deleted adenovirus vectors that does not require helper adenovirus. *Gene Ther,* 2001, vol. 8, 846-854
- **KUBO S ; MITANI K.** A new hybrid system capable of efficient lentiviral vector production and stable gene transfer mediated by a single helper-dependent adenoviral vector. *J Virol,* 2003, vol. 77, 2964-2971
- **SCHIEDNER G ; HERTEL S ; KOCHANEK S.** Efficient transformation of primary human amniocytes by E1 functions of Ad5: generation of new cell lines for adenoviral vector production. *Hum Gene Ther,* 2000, vol. 11, 2105-2116
- **FALLAUX FJ et al.** New helper cells and matched early region 1-deleted adenovirus vectors prevent generation of replication-competent adenoviruses. *Hum Gene Ther,* 1998, vol. 9, 1909-1917
- **GRAHAM FL.** Growth of 293 cells in suspension culture. *J Gen Virol,* 1987, vol. 68 (3), 937-940
- **SAKHUJA K et al.** Optimization of the generation and propagation of gutless adenoviral vectors. *Hum Gene Ther,* 2003, vol. 14, 243-254
- **THORPE HM ; WILSON SE ; SMITH MC.** Control of directionality in the site-specific recombination system of the Streptomyces phage phiC31. *Mol Microbiol,* 2000, vol. 38, 232-241
- **GROTH AC.** Olivares EC, Thyagarajan B, Calos MP. A phage integrase directs efficient site-specific integration in human cells. *Proc Natl Acad Sci U S A,* 2000, vol. 97, 5995-6000
- **ANDREAS S et al.** Enhanced efficiency through nuclear localization signal fusion on phage PhiC31-integrase: activity comparison with Cre and FLPe recombinase in mammalian cells. *Nucleic Acids Res,* 2002, vol. 30, 2299-2306
- **ALBA R. MM ; RODRIGUEZ E. ; GIL E. ; CALOS M.P ; BOSCH F. ; CHILLÓN M.** Generation of gutless helper free adenovirus using unidirectional FC31 recombinase as gene therapy vectors. *Gene Ther,* 2004, vol. 11, 164
- **EHRHARDT A ; KAY MA.** A new adenoviral helper-dependent vector results in long-term therapeutic levels of human coagulation factor IX at low doses in vivo. *Blood,* 2002, vol. 99, 3923-3930

- **MIAN A et al.** Long-term correction of ornithine transcarbamylase deficiency by WPRE-mediated overexpression using a helper-dependent adenovirus. *Mol Ther,* 2004, vol. 10, 492-499
- **GILBERT R et al.** Improved performance of a fully gutted adenovirus vector containing two full-length dystrophin cDNAs regulated by a strong promoter. *Mol Ther,* 2002, vol. 6, 501-509
- **ZOU L ; ZHOU H ; PASTORE L ; YANG K.** Prolonged transgene expression mediated by a helper-dependent adenoviral vector (hdAd) in the central nervous system. *Mol Ther,* 2000, vol. 2, 105-113
- **OSHIMA Y et al.** Intraocular gutless adenoviral-vectored VEGF stimulates anterior segment but not retinal neovascularization. *J Cell Physiol,* 2004, vol. 199, 399-411
- **KOEHLER DR et al.** Protection of Cftr knockout mice from acute lung infection by a helper-dependent adenoviral vector expressing Cftr in airway epithelia. *Proc Natl Acad Sci U S A,* 2003, vol. 100, 15364-15369
- **BILBAO R et al.** Comparison of high-capacity and first-generation adenoviral vector gene delivery to murine muscle in utero. *Gene Ther,* 2005, vol. 12, 39-47
- **OSTAPCHUK P ; HEARING P.** Minimal cis-acting elements required for adenovirus genome packinging. *J. Virol.,* vol. 77, 5127-5135
- **THORPE et al.** In vitro site-specific integration of bacteriophage DNA catalyzed by a recombinase of the resolvase/invertase family. *Proc Natl Acad Sci U S A,* 1998, vol. 5, 5505-5510
- **HOESS RH ; ABREMSKI K.** Interaction of the bacteriophage P1 Recombinase Cre with the recognition site loxP. *Proc Natl Acad Sci U S A,* 1984, vol. 81 (4), 1026-1029
- **BOUSSIF O. ; LEZOUALC'H F. ; ZANTA M.A. ; MERGNY M.D. ; SCHERMAN D. ; DEMENEIX B. ; BEHR J.P.** A versatile vector for gene and oligonucleotide transfer into cells in culture and in vivo: polyethylenimine. *Proc Natl Acad Sci U S A,* 1995, vol. 92 (16), 7297-7301
- **HEARING P ; SAMULSKI RJ ; WISHART WL ; SHENK T.** Identification of a repeated sequence element required for efficient encapsidation of the adenovirus type 5 chromosome. *J Virol,* 1987, vol. 61, 2555-2558
- **LOONSTRA A et al.** Growth inhibition and DNA damage induced by Cre recombinase in mammalian cells. *Proc Natl Acad Sci U S A,* 2001, vol. 98, 9209-9214

SEQUENCE LISTING

<110> UNIVERSITAT AUTONOMA DE BARCELONA

<120> METHOD FOR PRODUCING ADENOVIRUS VECTORS FOR GENE THERAPY AND DNA SEQUENCES USED THEREFOR

<130> CO-13456

<150> ES200601136
<151> 2006-04-28

<160> 16

<210> 1
<211> 34
<212> DNA
<213> Streptomyces

<220>
<221> Recombinase recognition sequence
<223> Minimal recognition sequence for PhiC31 recombinase in the bacterial genome

<400>
gtgccagggc gtgcccttgg gctccccggg cgcg 34

<210> 2
<211> 53
<212> DNA
<213> Streptomyces

<220>
<221> Recombinase recognition sequence
<223> Minimal attB sequence of the invention

<400>
ccgcggtgcg ggtgccaggg cgtgcccttg ggctccccgg gcgcgtactc 50
cac 53

<210> 3
<211> 84
<212> DNA
<213> phiC31 bacteriophage

<220>
<221> Recombinase recognition sequence
<223> Recognition sequence for phiC31 recombinase in the bacteriophage genome

<400>
agaagcggtt ttcgggagta gtgccccaac tggggtaacc tttgagttct 50
ctcagttggg ggcgtagggt cgccgacatg acac 84

<210> 4
<211> 218
<212> DNA
<213> phiC31 bacteriophage
<
<220>

<221> Recombinase recognition sequence
<222> 72..155
<223> Recognition sequence for phiC31 recombinase in the genome of phiC31 bacteriophage

<220>
<223> Recognition sequence for phiC31 recombinase in the genome of phiC31 bacteriophage and flanking sequences

<400>
actgacggac acaccgaagc cccggcggca accctcagcg gatgccccgg 50
ggcttcacgt tttcccaggt cagaagcggt tttcgggagt agtgccccaa 100
ctggggtaac ctttgagttc tctcagttgg gggcgtaggg tcgccgacat 150
gacacaaggg gttgtgaccg gggtggacac gtacgcgggt gcttacgacc 200
gtcagtcgcg cgagcgcg 218

<210> 5
<211> 21
<212> DNA
<213> Artificial sequence

<220>
<221> Primer
<223> 6600R2440

<400>
cacctcccag atccttatcg a 21

<210> 6
<211> 20
<212> DNA
<213> Artificial sequence

<220>
<221> Primer
<223> SP6

<400>
atttaggtga cactatagaa 20

<210> 7
<211> 20
<212> DNA
<213> Artificial sequence

<220>
<221> Primer
<223> T7

<400>
taatacgact cactataggg 20

<210> 8
<211> 21
<212> DNA
<213> Artificial sequence

<220>
<221> Primer

<223> Ad5140-160

<400>
cggaacacat gtaagcgacg g 21

<210> 9
<211> 25
<212> DNA
<213> Artificial sequence

<220>
<221> Recognition sequence for restriction endonuclease
<222> 2..7
<223> Recognition sequence for SgrAI

<220>
<221> Oligonucleotide
<223> Oligonucleotide for mutagenesis: MutDIRAgel

<400>
caccggtgta cacaggaagt gacaa 25

<210> 10
<211> 25
<212> DNA
<213> Artificial sequence

<220>
<221> Recognition sequence for restriction endonuclease
<222> 2..7
<223> Recognition sequence for SgrAI

<220>
<221> Oligonucleotide
<223> Oligonucleotide for mutagenesis: MutREVAgel

<400>
caccggtgta cacaccaaaa acgtc 25

<210> 11
<211> 83
<212> DNA
<213> Artificial sequence
<220>
<221> Recognition sequence for restriction endonuclease
<222> 15..20
<223> Recognition sequence for AgeI

<220>
<221> Recombinase recognition sequence
<222> 21..73
<223> attB sequence

<220>
<221> Recognition sequence for restriction endonuclease
<222> 74..81
<223> Recognition sequence for NotI

<220>
<221> Primer

<223> Oligonucleotide sequencing primer: attB_dir

<400>
ttataaaggt acccaccggt ccgcggtgcg ggtgccaggg cgtgcccttg 50
ggctccccgg gcgcgtactc cacgcggccg cat 83

<210> 12
<211> 78
<212> DNA
<213> Artificial sequence

<220>
<221> Recognition sequence for restriction endonuclease
<222> 9..14
<223> Recognition sequence for AgeI

<220>
<221> Recognition sequence for restriction endonuclease
<222> 16..23
<223> Recognition sequence for NotI

<220>
<221> Recombinase recognition sequence
<222> 24..57
<223> loxP sequence

<220>
<221> Recognition sequence for restriction endonuclease
<222> 58..65
<223> Recognition sequence for NotI

<220>
<221> Primer
<223> Oligonucleotide sequencing primer: attBloxP_rev

<400>
ttataaacac cggtcgcggc cgcataactt cgtataatgt atgctatacg 50
aagttatgcg gccgcgtgga gtacgcgc 78

<210> 13
<211> 78
<212> DNA
<213> Artificial sequence

<220>
<221> Recognition sequence for restriction endonuclease
<222> 9..16
<223> Recognition sequence for NotI

<220>
<221> Recombinase recognition sequence
<222> 18..51
<223> loxP sequence

<220>
<221> Recognition sequence for restriction endonuclease
<222> 52..57
<223> Recognition sequence for AvrII

<220>
<221> Oligonucleotide

<223> Oligonucleotide for introducing the loxP sequence:
loxP NotI/AvrII DIR(peGFP-C1)

<400>
tagcgaatgc ggccgctata acttcgtata gcatacatta tacgaagtta 50
tcctaggtga ctgggcacaa cagacaat 78

<210> 14
<211> 42
<212> DNA
<213> Artificial sequence

<220>
<221> Recognition sequence for restriction endonuclease
<222> 9..16
<223> Recognition sequence for NotI

<220>
<221> Recognition sequence for restriction endonuclease
<222> 18..23
<223> Recognition sequence for AvrII

<220>
<221> Oligonucleotide
<223> Oligonucleotide for introducing the loxP sequence:
NotI/AvrII REV(peGFP-C1)

<400>
gcatatcagc ggccgctcct agggtatcga cagagtgcca gc 42

<210> 15
<211> 481
<212> DNA
<213> Artificial sequence

<220>
<221> ITR
<222> 1..103
<223> Inverted terminal repeat of the 5' region of human adenovirus serotype 5

<220>
<221> Recombinase recognition sequence
<222> 194..247
<223> attB sequence of phiC31 recombinase

<220>
<221> Recombinase recognition sequence
<222> 253..288
<223> loxP sequence

<220>
<221> Packaging signal
<222> 308..481
<223> First 174 nucleotides of the 5' region of the Y packaging sequence human adenovirus serotype 5

<220>
<223> Sequence of the 5' region of the genome of adenovirus Ad5/loxP.phiC31

<400>
catcatcaat aatatacctt attttggatt gaagccaata tgataatgag 50
ggggtggagt ttgtgacgtg gcgcggggcg tgggaacggg gcgggtgacg 100
tagtagtgtg gcggaagtgt gatgttgcaa gtgtggcgga acacatgtaa 150
gcgacggatg tggcaaaagt gacgtttttg gtgtgtacac cggtccgcgg 200
tgcgggtgcc agggcgtgcc cttgggctcc ccgggcgcgt actccacgcg 250
gccgctataa cttcgtatag catacattat acgaagttat cctaggagcg 300
gccgcgaccg gtgtacacag gaagtgacaa ttttcgcgcg gttttaggcg 350
gatgttgtag taaatttggg cgtaaccgag taagatttgg ccattttcgc 400
gggaaaactg aataagagga agtgaaatct gaataatttt gtgttactca 450
tagcgcgtaa tatttgtcta gggccgcggg g 481

<210> 16
<211> 506
<212> DNA
<213> Artificial sequence

<220>
<221> ITR
<222> 26..128
<223> Inverted terminal repeat of the 5' region of human adenovirus serotype 5

<220>
<221> Recombinase recognition sequence
<222> 219..272
<223> attB sequence of phiC31 recombinase

<220>
<221> Recombinase recognition sequence
<222> 281..313
<223> loxP sequence

<220>
<221> Packaging signal
<222> 333..506
<223> First 174 nucleotides of the 5' region of the Y packaging sequence human adenovirus serotype 5

<220>
<223> Sequence of the region of plasmid PkP1.4DCMVDMCSattBloxPYGFPattP containing an attB sequence

<400>
gaattggatc cgaattctta attaacatca tcaataatat accttatttt 50
ggattgaagc caatatgata atgagggggt ggagtttgtg acgtggcgcg 100
gggcgtggga acggggcggg tgacgtagta gtgtggcgga agtgtgatgt 150
tgcaagtgtg gcggaacaca tgtaagcgac ggatgtggca aaagtgacgt 200
tttggtgtg tacaccggtc cgcggtgcgg gtgccagggc gtgcccttgg 250
gctccccggg cgcgtactcc acgcggccgc tataacttcg tatagcatac 300
attatacgaa gttatcctag gagcggccgc gaccggtgta cacaggaagt 350
gacaattttc gcgcggtttt aggcggatgt tgtagtaaat ttgggcgtaa 400
ccgagtaaga tttggccatt ttcgcgggaa aactgaataa gaggaagtga 450
aatctgaata attttgtgtt actcatagcg cgtaatattt gtctagggcc 500
gcgggg 506